(19) Europäisches Patentamt
European Patent Office
Office européen des brevets

(11) EP 3 931 585 B1

(12) **EUROPÄISCHE PATENTSCHRIFT**

(45) Veröffentlichungstag und Bekanntmachung des Hinweises auf die Patenterteilung:
**05.11.2025 Patentblatt 2025/45**

(21) Anmeldenummer: **19818001.0**

(22) Anmeldetag: **05.12.2019**

(51) Internationale Patentklassifikation (IPC):
***G01R 33/36*** *(2006.01)* ***G01R 33/34*** *(2006.01)*
***G01R 33/3415*** *(2006.01)*

(52) Gemeinsame Patentklassifikation (CPC):
**G01R 33/3664; G01R 33/3635;** G01R 33/34076; G01R 33/3415

(86) Internationale Anmeldenummer:
**PCT/EP2019/083857**

(87) Internationale Veröffentlichungsnummer:
**WO 2020/173590 (03.09.2020 Gazette 2020/36)**

(54) **MR-SPULENANORDNUNG MIT MEHREREN DIPOLANTENNEN UND VERBINDUNGSELEMENTEN MIT SPERRKREISEN**

MR COIL ASSEMBLY WITH MULTIPLE DIPOLE ANTENNAS AND CONNECTION ELEMENTS WITH BLOCKING CIRCUITS

ASSEMBLAGE DE BOBINE DE RÉSONANCE MAGNÉTIQUE COMPORTANT PLUSIEURS ANTENNES DIPOLAIRES ET ÉLÉMENTS DE LIAISON POURVUS DE FILTRES D’ARRÊT

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **27.02.2019 DE 102019105021**

(43) Veröffentlichungstag der Anmeldung:
**05.01.2022 Patentblatt 2022/01**

(73) Patentinhaber: **Forschungszentrum Jülich GmbH**
**52425 Jülich (DE)**

(72) Erfinder:
- **HONG, Suk Min**
  **52146 Würselen (DE)**
- **SHAH, Nadim Joni**
  **52428 Jülich (DE)**
- **CHOI, Chang-Hoon**
  **52428 Jülich (DE)**
- **FELDER, Jörg**
  **52428 Jülich (DE)**

(74) Vertreter: **Paul & Albrecht Patentanwälte PartG mbB**
**Stresemannallee 4b**
**41460 Neuss (DE)**

(56) Entgegenhaltungen:
**US-A- 5 202 635** **US-A1- 2010 253 333**

- **SUK-MIN HONG ET AL: "Design and simulation of dual-band dipole antenna for 1H/31P at 9.4T MRI", PROCEEDINGS OF THE INTERNATIONAL SOCIETY FOR MAGNETIC RESONANCE IN MEDICINE, ISMRM, JOINT ANNUAL MEETING ISMRM-ESMRMB, PARIS, FRANCE, 16-21 JUNE 2018, no. 4400, 1 June 2018 (2018-06-01), XP040703608**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Processed by Luminess, 75001 PARIS (FR)

## Beschreibung

Die vorliegende Erfindung betrifft eine Spulenanordnung zur Verwendung als Sende- und/oder Empfangsspule in einem MR-System, insbesondere MRT- und/oder MRS- System, die eine Dipolantennenanordnung mit mehreren über Verbindungselemente miteinander verbundenen Dipolantennen umfasst, wobei die Verbindungselemente ausgebildet sind, um von einem elektrisch verbindenden in einen elektrisch trennenden Zustand überführt zu werden, und umgekehrt, und die Anordnung derart getroffen ist, dass die Dipolantennen im elektrisch verbindenden Zustand der Verbindungselemente zumindest einen Teil einer bevorzugt zylindrischen Volumenspule und/oder eine mindestens eine Leiterschleife umfassende, insbesondere flache Leiterschleifenanordnung der Spulenanordnung bilden. Darüber hinaus betrifft die Erfindung ein MR-System, insbesondere MRT- und/oder MRS-System, mit einer solchen Spulenanordnung. Außerdem betrifft die Erfindung eine Verwendung einer solchen Spulenanordnung.

Die Magnetresonanztomographie, kurz MRT, wird seit vielen Jahren in der medizinischen Diagnostik angewandt. Bei der MRT handelt es sich um ein bildgebendes Verfahren mittels dem insbesondere die Struktur und Funktion von Gewebe und Organen in Form von Schnittbildern darstellbar ist. Die MRT kann auch als MRI bezeichnet werden, was für "Magnetic Resonance Imaging" steht. Die MRT baut auf den Wirkprinzipien der Kernspinresonanz, kurz NMR, insbesondere der Feldgradienten-NMR, auf und ist daher auch als Kernspintomographie bekannt. Der Begriff NMR ist ein Synonym für den Begriff Magnetresonanz, kurz MR. Die Abkürzung MRS steht für Magnetresonanz-Spektroskopie. Ein MR-System umfasst in der Regel einen insbesondere supraleitenden Magneten mit einer beispielsweise zylinderförmigen Spulenanordnung, der ausgebildet und/oder eingerichtet ist, um ein statisches, bevorzugt homogenes, Magnetfeld $B_0$ zu erzeugen, welches bevorzugt entlang der Achse der zylinderförmigen Spulenanordnung orientiert ist. Innerhalb einer zentralen Öffnung des Magneten befindet sich eine Hochfrequenz-Spulenanordnung, die einen Aufnahmeraum umgibt, in dem während einer MR-Untersuchung ein zu untersuchendes Medium, insbesondere eine Person oder ein Körperteil, beispielsweise der Kopf einer Person, angeordnet ist, um mit der Hochfrequenz-Spulenanordnung physikalisch wechselzuwirken. Die Hochfrequenz-Spulenanordnung kann sowohl zum Senden und Empfangen als auch als dedizierte Sende- oder Empfangs-Spulenanordnung ausgelegt werden. Bei der folgenden Beschreibung der Wirkprinzipien ist von einzelnen Kernspins die Rede. Dies stellt die klassische Beschreibung der quantenmechanischen Vorgänge dar. Indem das zu untersuchende Medium dem Magnetfeld $B_0$ ausgesetzt ist, werden Kernspins von Atomkernen des Mediums in Richtung des Magnetfelds $B_0$ ausgerichtet, d.h. sie erfahren eine longitudinale Magnetisierung in Richtung des Magnetfelds $B_0$. Mittels der Hochfrequenz-Spulenanordnung wird nun ein Hochfrequenz-Anregungsimpuls in Form eines zeitlich begrenzten hochfrequenten elektromagnetischen Wechselfelds erzeugt, dem das zu untersuchende Medium ausgesetzt wird, so dass bestimmte Atomkerne des zu untersuchenden Mediums durch das hochfrequente elektromagnetische Wechselfeld angeregt werden. Hierzu weist das hochfrequente elektromagnetische Wechselfeld eine Frequenz auf, die mit der Lamorfrequenz $f_L$ der anzuregenden Atomkerne in Resonanz ist. Die Lamorfrequenz $f_L$ ist durch die folgende Formel gegeben:

$$f_L = (\gamma B_0)/2\pi$$

Hierbei ist $\gamma$ die gyromagnetische Konstante, die für eine vorgegebene Kernsorte konstant ist. $B_0$ ist das statische Magnetfeld.

Anregung von Atomkernen bedeutet, dass die Magnetisierung der Kernspins aus der Richtung des $B_0$-Feldes, also aus der Gleichgewichtslage, ausgelenkt, d.h. aus der Richtung des $B_0$-Feldes gekippt wird. Somit wird die longitudinale Magnetisierung der Kernspins zumindest teilweise in eine um die Feldrichtung des $B_0$-Feldes präzidierende Transversalmagnetisierung umgewandelt. Hierbei präzidieren die Kernspins mit ihrer Lamorfrequenz. Die präzidierende Transversalmagnetisierung induziert schließlich eine hochfrequente elektrische Wechselspannung in einer kombinierten Hochfrequenz-Sende- und Empfangs-Spulenanordnung oder einer dedizierten Hochfrequenz-Empfangs-Spulenanordnung, deren Frequenz insbesondere der Lamorfrequenz der angeregten und zu beobachtenden oder zu detektierenden Atomkerne entspricht. Hierbei gibt die Amplitude der induzierten Wechselspannung die Stärke der Transversalmagnetisierung an. Bei der MRT geht es darum, Schnittbilder der räumlichen Verteilung der Transversalmagnetisierung und/oder Resonanzspektren zu erzeugen und darzustellen. Hierfür ist es notwendig, die klassischen NMR-Signale bestimmten räumlichen Abschnitten des zu untersuchenden Mediums zuzuordnen, also eine Ortskodierung vorzunehmen. Dies geschieht bei der MRT mit Hilfe von zeitlich veränderlichen magnetischen Gradientenfeldern, die von Gradientenspulen erzeugt werden, die in der Öffnung des $B_0$-Feld-Magneten angeordnet sind.

Bei der klassischen MRT basiert die Bildgebung auf der Detektion von $^1$H-Kernen, also Wasserstoffatomkernen. Die klinische MRT-Bildgebung erfolgt üblicherweise mit einem $B_0$ im Bereich von etwa 0,1 bis 3,0 T. Insbesondere in der Forschung werden jedoch immer häufiger auch höhere Magnetfeldstärken eingesetzt. Mittels der sogenannten Ultrahochfeld-MRT, kurz UHF-MRT, die mit einem $B_0 \geq 7$ T arbeitet, lassen sich neben den $^1$H-Kernen auch andere Atomkerne, sogenannte X-Kerne, effizient detektieren. Hierbei steht das "X" für einen beliebigen Atomkern mit Kernspin, außer $^1$H.

Beispiele für X-Kerne sind insbesondere Kerne, wie etwa der $^{31}$P-Kern und der $^{23}$Na-Kern, die bei physiologischen Prozessen eine wichtige Rolle spielen. X-Kerne liefern aufgrund ihrer verglichen mit $^{1}$H-Kernen deutlich geringeren Konzentration und ihrer physikalischen Eigenschaften normalerweise ein deutlich schwächeres Signal als $^{1}$H-Kerne, deren Signal auch als Protonensignal bezeichnet wird. Bei der UHF-MRT lässt sich jedoch insbesondere für die X-Kerne ein vergleichsweise hohes Signal-Rausch-Verhältnis, ein vergleichsweise hoher Phasenkontrast und eine vergleichsweise hohe Spektralauflösung realisieren.

Die Bestrebungen gehen daher dahin, eine Hochfrequenz-Spulenanordnung zu schaffen, die für eine sogenannte $^{1}$H/X-Kern-Anwendung geeignet ist, d.h. dazu in der Lage ist, verschiedene Atomkerne anzuregen und/oder zu detektieren oder zu beobachten. Die Spulenanordnung muss also sowohl für $^{1}$H-Kerne als auch für mindestens einen X-Kern sensitiv sein. Konkret muss die Spulenanordnung in der Lage sein, einerseits ein hochfrequentes, elektromagnetisches Wechselfeld, das mit der Lamorfrequenz $f_L$ der $^{1}$H-Kerne in Resonanz ist, und andererseits ein hochfrequentes, elektromagnetisches Wechselfeld, das mit der Lamorfrequenz $f_L$ einer ausgewählten Sorte von X-Kernen in Resonanz ist, abzustrahlen beziehungsweise aufzunehmen. Die Spulenanordnung ist dann doppelt-abgestimmt, nämlich auf zwei Resonanzfrequenzen, und/oder doppelt resonant.

Unterschiedliche Möglichkeiten zur Abstimmung von Antennen oder Spulenanordnungen mit Antennen auf bestimmte Frequenzen sind seit langem bekannt.

So offenbart die US2,229,865 eine Zwei-Band-Dipolantenne beziehungsweise eine doppelt resonante Dipolantenne, deren elektrische Länge durch Sperrkreise variiert werden kann. Die Dipolantenne erzeugt die Doppelresonanz durch die Einschränkung von Stehwellen zwischen den Sperrelementen. Demgegenüber erzeugt die gesamte Länge der Dipolantenne ihren natürlichen Stehwellenstrom. Die höhere Resonanz benutzt also nur einen Teil der physikalischen Leiterstruktur. Die benötigte Länge der Dipolantenne, um diese bei der Detektierung von X-Kernen einsetzen zu können, beträgt beispielsweise ungefähr 125 cm für $^{31}$P-Kerne bei $B_0$=7 T und ungefähr 187 cm für $^{23}$Na-Kerne bei $B_0$=7 T. Die Verwendung einer monofrequenten Dipolbeziehungsweise Monopolantennenanordnung bei der Detektierung von 1H-Kernen wird von A.J.E. Raaijmakers u.a. in dem Aufsatz "Design of a radiative surface coil array element at 7T: the single-side adapted dipole antenna", Magn. Reson. Med. Off. J. Soc. Magn. Reson. Med. Soc. Magn. Reson. Med., Bd. 66, Nr. 5, S. 1488-1497, Nov. 2011, und von Hong Suk-Min, Park Joshua Haekyun, Woo Myung-Kyun, Kim Young-Bo, und Cho Zang-Hee in dem Aufsatz "New design concept of monopole antenna array für UHF 7T MRI", Magn. Reson. Med., Bd. 71, Nr. 5, S. 1944-1952, Juli 2013 untersucht. Hierbei beträgt die benötigte Länge einer Dipolantenne beispielsweise ungefähr 50 cm für $^{1}$H-Kerne bei $B_0$=7 T oder ungefähr 37 cm für $^{1}$H-Kerne bei $B_0$=9,4 T. Diese Länge liegt nahe an einer für Kopfuntersuchungen geeigneten Abmessung und bietet eine relativ große Durchdringungstiefe und gegenüber Anordnungen aus Leiterschleifen symmetrischere MRT relevante Feldkomponentenmuster. Aufgrund der für die Detektierung von X-Kernen benötigten relativ großen Länge sind die vorbekannten Zwei-Band-Dipolantennen jedoch nicht geeignet, um beispielsweise bei MRT-Untersuchungen des Kopfes verwendet werden zu können.

Des Weiteren sind Spulenanordnungen bekannt, die eine sogenannte Volumenspule umfassen oder daraus bestehen. Ein Beispiel für einen Volumenspule ist die Alderman-Grant-Spule. Diese wurde von D.W. Aldermann und D.M. Grant in dem Aufsatz "An efficient decoupler coil design which reduces heating in conductive samples in superconducting spectrometers", J. Magn. Reson. 1969, Bd. 36, Nr. 3, S. 447-451, Dez. 1979, beschrieben. Sie ist in der Lage ein relativ homogenes Magnetfeld zu erzeugen und ist gut geeignet für hohe Feldstärken. Die Alderman-Grant-Spule besteht aus 2 oder 4 Stäben, die an ihren Enden jeweils über einen Endring verbunden sind. Ihr Wirkungsprinzip beruht auf der Verwendung symmetrischer Streifenleitungen und geht auf das Konzept des "Slotted Tube Resonators" (Schneider und Dullenkopf, 1977) zurück. Ein weiteres Beispiel für eine Volumenspule ist die sogenannte Birdcage-Spule, insbesondere eine vier- bis achtfüßige Birdcage-Spule, die ebenfalls Ringe und Stäbe umfassen kann.

Beispielsweise wird von J.R. Fitzsimmons, B.L. Beck, und H. Ralph Brooker in dem Aufsatz "Double resonant quadrature birdcage", Magn. Reson. Med., Bd. 30, Nr. 1, S. 107-114, Frühjahr 1993, und von A.M. Hudson, W. Köckenberger, und R.W. Bowtell in dem Aufsatz "Dual resonant birdcage coils for 1H detected 13C microscopic imaging at 11.7T", Magma N.Y.N, Bd, 10, Nr. 2, S. 61-68, Juni 2000 jeweils eine konzentrische Anordnung von zwei physikalisch getrennten Birdcage-Spulen beschrieben. Hierbei ist eine Birdcage-Spule auf eine $^{1}$H-Kern-Resonanzfrequenz und die andere Birdcage-Spule auf eine X-Kern-Resonanzfrequenz abgestimmt. Diese Spulenanordnungen haben jedoch den Nachteil, dass sie für die obere Resonanzfrequenz ein gegenüber ihren monofrequenten Implementierungen ein stark reduziertes Signal-Rausch-Verhältnis aufweisen.

Es sind auch doppelt resonante beziehungsweise doppelt abgestimmte Birdcage-Spulen bekannt. So wird von Y. Duan, B.S. Peterson, F. Liu, T.R. Brown, T.S. Ibrahim, und A. Kangarlu in dem Aufsatz "Computational and experimental optimization of a double-tuned 1H/31P four-ring birdcage head coil für MRS at 3T", J.Magn. Reson. Imaging, Bd. 29, Nr. 1, S. 13-22, Frühjahr 2009, und von J. Murphyboesch, R. Srinivasan, L. Carvajal, und T.R. Brown in dem Aufsatz "Two Configurations of the Four-Ring Birdcage Coil for 1H Imaging and 1H-Decoupled 31P Spectroscopy of the Human Head", J. Magn. Reson. B, Bd. 103, Nr. 2, S. 103-114, Frühjahr 1994 jeweils eine doppelt resonante Birdcage-Spule, genauer gesagt eine 4-Ring-Birdcage-Spule, die durch Verdoppelung der Endringe gebildet wird, beschrieben. Eine weitere doppelt resonante Birdcage-Spule wird von G.B. Matson, P. Vermathen, und T.C. Hill in dem Aufsatz "A parctical double-tuned 1H/31P quadrature birdcage headcoil optimized for 31P operation", Magn. Reson. Med., Bd. 42, Nr. 1, S. 173-182, Frühjahr 1999 beschrieben. Bei der letztgenannten Birdcage-Spule sind alternierend, d.h. auf jedem zweiten Stab, $^{1}$H-Sperrkreise angebracht.

Werden die zuvor erwähnten doppelt resonanten Birdcage-Spulen bei der MRT, insbesondere der HF-MRT, eingesetzt, sind die Kapazitätswerte zur Abstimmung, insbesondere Feinabstimmung, auf die beiden Resonanzfrequenzen sehr klein. Dies hängt unter anderem mit dem hohen Induktivitätswert der Birdcage-Spule zusammen. Beispielsweise benötigt eine 8-stufige Hochpass-Birdcage-Spule mit einem Durchmesser von 26 cm und einer Länge von 20 cm eine Kapazität von 2pF. Eine 16-stufige Tiefpass-Birdcage-Spule mit gleichem Durchmesser und gleicher Länge benötigt 0,36 pF an Kapazität. Bei 300 MHz liegen die zur Abstimmung benötigten Kapazitätswerte in etwa bei 0,5pF. Die zuvor erwähnten Kapazitätswerte liegen im Bereich von üblichen Streukapazitäten und/oder Parasitärkapazitäten. Eine Berechnungsmethode für die Kapazitäten in einer Birdcage-Spule wird von C.-L. Chin, C. M. Collins, S. Li, B.J. Dardzinski, und M.B. Smith in dem Aufsatz "BirdcageBuilder: Design of Specified-Geometry Birdcage Coils with Desired Current Pattern and Resonant Frequency", Concepts Magn. Reson., Bd. 15, Nr. 2, S. 156-163, Juni 2002 beschrieben. Somit ist eine Abstimmung der vorbekannten doppelt resonanten Birdcage-Spulen nur äußerst schwierig möglich.

Außerdem wird in der US 5,462,055 eine Spulenanordnung zur Verwendung in einem kombinierten MRT/Hyperthermie-System offenbart. Die Spulenanordnung umfasst eine Dipolantennenanordnung mit mehreren über Verbindungselemente miteinander verbundenen Dipolantennen. Hierbei sind die Verbindungselemente ausgebildet, um von einem elektrisch verbindenden in einen elektrisch trennenden Zustand überführt zu werden, und umgekehrt. Konkret sind die Verbindungselemente als Transistorschaltungen ausgeführt, die einen Transistor umfassen, der durch Anlegen entsprechender Steuersignale, die von einer Steuerung bereitgestellt werden, geschaltet wird. Die Dipolantennen bilden im elektrisch verbindenden Zustand der Verbindungselemente einen Teil einer zylindrischen Volumenspule der Spulenanordnung. Durch Umschalten der Verbindungselemente zwischen den beiden Zuständen lässt sich mittels der Spulenanordnung entweder MRT-Anregungsenergie oder Hyperthermie-Energie bereitstellen. Genauer gesagt wird die Volumenspule für die Protonenbildgebung verwendet, während die Dipolantennen für die Hyperthermieanregung verwendet werden. Bei der Spulenanordnung der US 5,462,055 ist das Umschalten der Verbindungselemente zwischen dem elektrisch verbindenen und dem elektrisch trennenden Zustand relativ aufwendig, insbesondere weil eine zusätzliche Steuerung vorgesehen sein muss.

Das Dokument US 2010/0253333 A1 offenbart eine doppelt abgestimmte Volumenspule, die angepasst ist, um einen Endringmodus bereitzustellen.

Das Dokument US 5,202,635 offenbart einen Radiofrequenz-Volumenresonator für die Kernspintomografie.

Vor diesem Hintergrund liegt der vorliegenden Erfindung die Aufgabe zugrunde, eine Spulenanordnung der eingangs genannten Art bereitzustellen, die derart ausgebildet ist, dass sie verglichen mit den vorbekannten Spulenanordnungen mit geringerem Aufwand abgestimmt und betrieben werden kann.

Diese Aufgabe ist erfindungsgemäß gelöst durch ein Spulensystem nach Anspruch 1. Die Verbindungselemente umfassen Verbindungselement-Sperrkreise, die automatisch sperren, wenn eine hochfrequente Wechselspannung mit einer der Sperrfrequenz der Verbindungselement-Sperrkreise entsprechenden Frequenz an die Spulenanordnung angelegt wird. Die Verbindungselement-Sperrkreise sperren hierbei solange, wie die hochfrequente Wechselspannung der Sperrfrequenz an der Spulenanordnung anliegt. Bei der hochfrequenten Wechselspannung kann es sich auch um eine in der Spulenanordnung induzierte hochfrequente Wechselspannung handeln.

Der Erfindung liegt somit die Überlegung zugrunde, die Verbindungselemente einer Spulenanordnung mit Verbindungselement-Sperrkreisen zu versehen, die automatisch sperren, wenn eine hochfrequente Wechselspannung mit einer der Sperrfrequenz der Verbindungselement-Sperrkreise entsprechende Frequenz an die Spulenanordnung angelegt wird, ansonsten jedoch nicht oder zumindest nicht vollständig sperren. Hierbei ist die Sperrfrequenz die Frequenz, die den jeweiligen Verbindungselement-Sperrkreis nicht passieren kann. Wenn die Verbindungselement-Sperrkreise sperren, befinden sich die Verbindungselemente in ihrem elektrisch trennenden Zustand, was bedeutet, dass die Dipolantennen voneinander elektrisch getrennt sind. Die im elektrisch verbindenden Zustand der Verbindungselemente vorliegende Volumenspule und/oder Leiterschleifenanordnung ist -wenn die Sperrkreise sperren -dann sozusagen in einzelne Dipolantennen aufgetrennt, die insbesondere unabhängig voneinander arbeiten und/oder betrieben werden können. Eine Spule umfasst mindestens eine Leiterschleife, ist also im einfachsten Fall eine Leiterschleife. Die Leiterschleifenanordnung kann, insbesondere wenn sie flach ausgebildet ist, beispielsweise bei der Untersuchung einer Wirbelsäule eingesetzt werden. Eine Spulenanordnung mit einer flachen Leiterschleifenanordnung kann als Oberflächenspulenanordnung bezeichnet werden. Bei der Volumenspule kann es sich insbesondere um eine Alderman-Grant- oder Birdcage-Spule handeln. Dadurch, dass die erfindungsgemäße Spulenanordnung Verbindungselement-Sperrkreise verwendet, um die Dipolantennen automatisch, also frequenzabhängig, elektrisch miteinander zu verbinden oder voneinander zu trennen, kann die erfindungsgemäße Spulenanordnung verglichen mit den vorbekannten Spulenanordnungen mit geringerem Aufwand abgestimmt und betrieben werden und benötigt keine Ansteuerung der Verbindungselement-Sperrkreise. Im Gegensatz zu aktiven Schaltern, welche Verbindungen im Zeitbereich herstellen bzw. trennen, ermöglicht die Verwendung von frequenzselektiven Sperrkreisen vorteilhaft auch die Verwendung der hier beschriebenen Spulenanordnung in sogenannten Entkopplungsexperimenten unter Verwendung des Kern-Overhauser Effekts.

Erfindungsgemäß ist die Spulenanordnung derart ausgestaltet, dass die Dipolantennen im elektrisch trennenden Zustand der Verbindungselemente ein hochfrequentes, elektromagnetisches Wechselfeld mit einer ersten, insbesondere der Sperrfrequenz entsprechenden Frequenz abstrahlen und/oder aufnehmen, und dass die sich im elektrisch verbindenden Zustand der Verbindungselemente ergebende Volumenspule und/oder jede Leiterschleife der sich im elektrisch verbindenden Zustand der Verbindungselemente ergebenden Leiterschleifenanordnung ein hochfrequentes, elektromagnetisches Wechselfeld mit einer zweiten von der ersten verschiedenen Frequenz abstrahlt und/oder aufnimmt. Es liegt dann insbesondere eine doppelt abgestimmte Spulenanordnung vor. Die erfindungsgemäße Spulenanordnung ermöglicht, dass insbesondere in der Hochfeld-MRT zur Feinabstimmung der ersten und zweiten Abstrahlfrequenzen Kondensatoren mit Kapazitätswerten eingesetzt werden können, die deutlich größer und somit deutlich besser realisierbar als die aus dem Stand der Technik bekannten Kapazitätswerte sind. Darüber hinaus können Spulen mit deutlich leichter zu realisierenden Induktivitätswerten eingesetzt werden. Die erste Frequenz kann eine $^{1}$H-Kern-Resonanzfrequenz sein, was bedeutet, dass das von den Dipolantennen abgestrahlte und/oder aufgenommene hochfrequente, elektromagnetische Wechselfeld und/oder eine entsprechende in der Spulenanordnung induzierte hochfrequente elektrische Wechselspannung mit der Lamorfrequenz der 1H-Kerne für ein bestimmtes $B_0$-Feld in Resonanz ist. In diesem Fall werden für die $^{1}$H-Bildgebung Stehwellen zwischen den Verbindungselement-Sperrkreisen beziehungsweise innerhalb der einzelnen Dipolantennen gebildet. Die zweite Frequenz kann eine X-Kern-Resonanzfrequenz, insbesondere eine $^{31}$P-Kern- oder $^{23}$Na-Kern-Resonanzfrequenz, sein. Dies bedeutet, dass das von der Volumenspule oder jeder Leiterschleife der Leiterschleifenanordnung abgestrahlte und/oder aufgenommene hochfrequente, elektromagnetische Wechselfeld und/oder eine entsprechende in der Spulenanordnung induzierte hochfrequente elektrische Wechselspannung mit der Lamorfrequenz eines bestimmten X-Kerns für ein bestimmtes $B_0$-Feld in Resonanz ist. Die X-Kern-Resonanzfrequenz entspricht also der Lamorfrequenz der X-Kerne. Die Leiterschleifen können also jeweils als Antennen für die X-Kern-Resonanzfrequenz fungieren. Mittels der Spulenanordnung lassen sich also durch elektrisches Trennen oder Verbinden der Dipolantennen verschiedene elektrische Konfigurationen für die beiden Resonanzfrequenzen bereitstellen, die sich beide vorteilhaft für MRT-Untersuchungen verwenden lassen. Die erfindungsgemäße Spulenanordnung ist ohne signifikante Verluste für zwei MRT-Kerne sensibel, kommt insbesondere ohne Verwendung verlustbehafteter Bauteile aus. Daher weist die Spulenanordnung verglichen mit den vorbekannten Spulenanordnungen **eine hohe** Sensitivität, welche mit monofrequenten Anordnungen vergleichbar ist, und damit ein hohes Signal-Rausch-Verhältnis, SNR, und eine hohe Effizienz für beide Resonanzfrequenzen, also bei Aufnahmen von beiden Kernen, auf. Bei den Verbindungselement-Sperrkreisen handelt es sich vorzugsweise um sogenannte $^{1}$H-Verbindungselement-Sperrkreise, kurz $^{1}$H-Sperrkreise, deren Sperrfrequenz einer $^{1}$H-Kern-Resonanzfrequenz entspricht.

Generell gilt, dass für X-Kerne die Homogenität besser wird, je mehr Dipolantennen verwendet werden. Jedoch ist die Anzahl der Dipolantennen dadurch begrenzt, dass ab einer bestimmten Anzahl die Kopplung der Dipolantennen im $^{1}$H-Betrieb zu hoch ist, um diese sinnvoll einsetzen zu können. Es hat sich eine Anzahl von vier bis acht, insbesondere vier oder acht, Dipolantennen als vorteilhaft herausgestellt.

Vorteilhaft umfassen die Dipolantennen jeweils ein stabförmiges Basiselement, an dessen axial gegenüberliegenden Enden sich jeweils ein insbesondere ringsegmentartiges Leiterbahnsegment anschließt. Die axialen Enden des stabförmigen Basiselements schließen sich insbesondere mittig an das jeweilige Leiterbahnsegment an. Bevorzugt sind die Leiterbahnsegmente der Dipolantennen gleich groß. Im Falle von ringsegmentartigen Leiterbahnsegmenten, weisen diese insbesondere die gleiche Bogenlänge auf. Die stabförmigen Basiselemente der Dipolantennen können zumindest im Wesentlichen parallel zueinander angeordnet sein. Zweckmäßigerweise sind die stabförmigen Basiselemente der Dipolantennen in Umfangsrichtung der Volumenspule oder in Längsrichtung der Leiterschleifenanordnung gleichmäßig voneinander beabstandet angeordnet. Die Länge der stabförmigen Basiselemente der Dipolantennen, die Höhe der Volumenspule oder die Breite der Leiterschleifenanordnung kann insbesondere für Messungen am menschlichen Kopf im Bereich von 25 bis 30 cm liegen, insbesondere 25 cm oder 28 cm betragen. Vorzugsweise ist die Länge der Dipolantenne an die Sperrfrequenz der Verbindungselement-Sperrkreise angepasst. Maßnahmen zur Variation der physikalischen Länge von Dipolantennen z. B. durch Einbringen von konzentrierten Bauteilen sind dem Fachmann bekannt und unter anderem in G. Janzen, "Kurze Antennen. Entwurf und Berechnung verkürzter Sende- und Empfangsantennen", Kosmos Verlags GmbH, Juni 1989, ISBN: 978-3440054697, beschrieben.

Gemäß einer weiteren Ausgestaltung sind die Leiterbahnsegmente der Dipolantennen über die Verbindungselemente unter Bildung zweier Leiterbahnen, insbesondere zweier ringförmig geschlossener Leiterbahnen, miteinander verbunden. Insbesondere im Falle zweier ringförmig geschlossener Leiterbahnen kann die Anzahl an Verbindungselementen pro Leiterbahn der Anzahl an Dipolantennen entsprechen. Insbesondere im Falle einer flachen Leiterschleifenanordnung ist die Anzahl an Verbindungselementen pro Leiterbahn bevorzugt um eins geringer als die Anzahl an Dipolantennen. Der Durchmesser der ringförmig geschlossenen Leiterbahnen oder der Volumenspule kann insbesondere für Messungen am menschlichen Kopf im Bereich von 25 bis 30 cm liegen, insbesondere 26 cm betragen.

Gemäß einer weiteren vorteilhaften Ausführungsform umfasst zumindest einer der Verbindungselement-Sperrkreise eine Verbindungselement-Spule und einen Verbindungselement-Kondensator, die parallel geschaltet sind. Die Verbindungselement-Spule weist bevorzugt eine Induktivität im Bereich von 38 bis 41 nH, besonders bevorzugt im Bereich von 39 bis 40 nH, insbesondere von 39 nH oder 40 nH auf. Der Verbindungselement-Kondensator weist vorzugsweise eine Kapazität im Bereich von 6 bis 8 pF, insbesondere von 6,8 pF auf. Zumindest ein Verbindungselement kann einen zweiten Verbindungselement-Kondensator umfassen, der mit dem Verbindungselement-Sperrkreis in Reihe geschaltet ist. Der zweite Verbindungselement-Kondensator weist bevorzugt eine Kapazität im Bereich von 5 bis 50 pF, besonders bevorzugt im Bereich von 8,2 bis 40 pF, insbesondere von 8,2 pF oder 40 pF auf. Zweckmäßigerweise ist der zweite Verbindungselement-Kondensator ausgebildet, weist insbesondere eine geeignete Kapazität auf, um die Spulenanordnung auf die zweite Frequenz abzustimmen, insbesondere feinabzustimmen. Ein Kapazitätswert des zweiten Verbindungselement-Kondensators in der vorstehend genannten Größenordnung ist leicht realisierbar, was dazu beiträgt, dass sich die Spulenanordnung mit relativ geringem Aufwand abstimmen lässt. Die zuvor erwähnten Kapazitäts- und Induktivitätswerte sind insbesondere vorteilhaft bei einer Verwendung der Spulenanordnung als Teil eines MR-Systems, welches einen Magneten umfasst, der ausgebildet ist, um ein Magnetfeld $B_0$ von 7T zu erzeugen.

In bevorzugter Ausgestaltung sind die stabförmigen Basiselemente der Dipolantennen jeweils mittig unter Bildung zweier Pole der jeweiligen Dipolantenne aufgetrennt. Vorteilhafterweise sind die stabförmigen Basiselemente der Dipolantennen jeweils mit einer Anschlusseinrichtung zur Verbindung mit einer Wechselspannungsversorgungs- und/oder aufnahmeeinrichtung versehen, die mit den beiden Polen der Dipolantenne verbundene elektrische Anschlusselemente umfasst. Bevorzugt sind die stabförmigen Basiselemente jeweils in ihrem mittleren Abschnitt mit der Anschlusseinrichtung versehen. Über die elektrischen Anschlusselemente der Anschlusseinrichtungen kann jeweils ein $^1$H-Kern-Signal eingespeist werden, insbesondere die jeweilige Dipolantenne mit einer hochfrequenten Wechselspannung, deren Frequenz bevorzugt einer $^1$H-Kern-Resonanzfrequenz entspricht, gespeist werden, um zu bewirken, dass die Dipolantennen jeweils ein hochfrequentes, elektromagnetisches Wechselfeld mit der $^1$H-Kern-Resonanzfrequenz abstrahlen. Wenn die Frequenz der gespeisten hochfrequenten Wechselspannung der Sperrfrequenz der Verbindungselement-Sperrkreise entspricht, wird ein zu der gespeisten hochfrequenten Wechselspannung korrespondierender elektrischer Wechselstrom auf der jeweiligen Dipolantenne isoliert. Über die elektrischen Anschlusselemente der Anschlusseinrichtungen kann jeweils auch eine in der Spulenanordnung induzierte hochfrequente Wechselspannung abgegriffen und/oder aufgenommen werden.

Die Spulenanordnung kann derart ausgestaltet sein, dass eine Einspeisung und/oder ein Abgriff von $^1$H-Kern-Signalen und/oder X-Kern-Signalen über die elektrischen Anschlusselemente zumindest einer Anschlusseinrichtung, insbesondere aller Anschlusseinrichtungen, erfolgen kann. Hierbei umfasst der Begriff "$^1$H-Kern-Signale" sowohl eine in die Spulenanordnung eingespeiste hochfrequente Wechselspannung, deren Frequenz der $^1$H-Kern-Resonanzfrequenz entspricht, als auch eine an der Spulenanordnung abgegriffene, aufgrund angeregter $^1$H-Kerne in der Spulenanordnung induzierte hochfrequente elektrische Wechselspannung, deren Frequenz insbesondere der $^1$H-Kern-Resonanzfrequenz entspricht. Der Begriff "X-Kern-Signale" umfasst sowohl eine in die Spulenanordnung eingespeiste hochfrequente Wechselspannung, deren Frequenz sich von der $^1$H-Kern-Resonanzfrequenz unterscheidet und bevorzugt einer X-Kern-Resonanzfrequenz entspricht, als auch eine an der Spulenanordnung abgegriffene, aufgrund angeregter X-Kerne in der Spulenanordnung induzierte hochfrequente elektrische Wechselspannung, deren Frequenz insbesondere der X-Kern-Resonanzfrequenz entspricht.

Vorteilhaft ist die Spulenanordnung derart ausgestaltet, dass sie in einem 4-Kanal- und/oder 2-Kanal-Quadraturbetrieb betrieben werden kann. Beim 4-Kanal-Quadraturbetrieb erfolgt die Einspeisung/der Abgriff des X-Kern-Signals beziehungsweise des 1H-Kern-Signals an vier Anschlusseinrichtungen, wohingegen beim 2-Kanal-Quadraturbetrieb die Einspeisung/der Abgriff des X-Kern-Signals beziehungsweise des 1H-Kern-Signals an zwei Anschlusseinrichtungen erfolgt. Der Quadraturbetrieb ermöglicht die Erzeugung **eines zirkular** polarisierten Feldes. Durch die Erzeugung einer zirkularen Feldpolarisation wird die benötigte Sendeleistung reduziert und das Empfangs-SNR verglichen mit linearer Feldpolarisation erhöht.

Gemäß einer bevorzugten Ausgestaltung ist die Spulenanordnung derart ausgestaltet, dass eine Einspeisung und/oder ein Abgriff von X-Kern-Signalen über die elektrischen Anschlusselemente eines Paares aus zwei benachbarten, insbesondere in Umfangsrichtung der Volumenspule benachbarten, Anschlusseinrichtungen von insbesondere insgesamt vier Anschlusseinrichtungen erfolgen kann. Die Spulenanordnung kann derart ausgestaltet sein, dass die sich im elektrisch verbindenden Zustand der Verbindungselemente ergebende Volumenspule und/oder Leiterschleifenanordnung über die beiden benachbarten Anschlusseinrichtungen in einem Quadraturbetrieb mit einer hochfrequenten Wechselspannung, deren Frequenz sich von der $^1$H-Kern-Resonanzfrequenz unterscheidet und bevorzugt einer X-Kern-Resonanzfrequenz entspricht, gespeist werden kann, um zu bewirken, dass die Volumenspule oder jede Leiterschleife der Leiterschleifenanordnung ein hochfrequentes elektromagnetisches Wechselfeld mit einer X-Kern-Resonanzfrequenz abstrahlt. Hierbei können die beiden benachbarten Anschlusseinrichtungen mit um 90 Grad phasenverschobenen Signalen angesteuert werden. Im Falle einer Speisung an vier Anschlusseinrichtungen, welche in der MRT als "four port drive" bekannt ist und ebenfalls die Erzeugung einer zirkular polarisierten Feldverteilung ermöglicht, ist die Phasenbeziehung zwischen den Speisesignalen bevorzugt 0 -90 -180 -270 Grad.

Falls sich im elektrisch verbindenden Zustand der Verbindungselemente eine Leiterschleifenanordnung ergibt, kann eine Einspeisung und/oder ein Abgriff von $^{1}$H-Kern-Signalen und/oder X-Kern-Signalen auch an den freien Enden der beiden Leiterbahnen erfolgen.

In der Mitte einer Dipolantenne zwischen den beiden Polen kann ein Dipol-Kondensator vorgesehen sein, der insbesondere Teil der Anschlusseinrichtung ist. Der Begriff "Dipol-Kondensator" dient hier lediglich der Unterscheidung gegenüber den anderen Kondensatoren, wobei dem Präfix "Dipol-"keine funktionale Bedeutung zukommt. Der Dipol-Kondensator weist bevorzugt eine Kapazität von 0 pF auf. Die Dipol-Kondensatoren können auch Kapazitätswerte abweichend von 0 pF aufweisen, um eine verbesserte Impedanzanpassung insbesondere an eine angeschlossene Übertragungsleitung zu erzielen. Zweckmäßigerweise ist der Dipol-Kondensator ausgebildet, weist insbesondere eine geeignete Kapazität auf, um die Spulenanordnung auf die erste Frequenz abzustimmen, insbesondere feinabzustimmen.

Gemäß einer weiteren Ausgestaltung ist ein insbesondere parallel zu dem Dipol-Kondensator geschaltetes Koppelelement vorgesehen, das mit den beiden Polen der Dipolantenne elektrisch verbunden ist und diese überbrückt. Hierbei ist das Koppelelement ausgebildet, um von einem elektrisch verbindenden in einen elektrisch trennenden Zustand überführt zu werden, und umgekehrt. Das Koppelelement ist bevorzugt Teil der Anschlusseinrichtung. Bevorzugt umfasst das Koppelelement einen Koppelelement-Sperrkreis, der automatisch sperrt, wenn eine hochfrequente Wechselspannung mit einer der Sperrfrequenz des Koppelelement-Sperrkreises entsprechenden Frequenz an die Spulenanordnung, insbesondere an die elektrischen Anschlusselemente der Anschlusseinrichtung der entsprechenden Dipolantenne, angelegt oder darin induziert wird. Hierbei entspricht die Sperrfrequenz des Koppelelement-Sperrkreises insbesondere der ersten Frequenz. Die Sperrfrequenzen der Verbindungselement-Sperrkreise und der Koppelelement-Sperrkreise stimmen vorzugsweise überein. Der Koppelelement-Sperrkreis sperrt solange, wie die hochfrequente Wechselspannung der Sperrfrequenz an der Spulenanordnung anliegt.

Vorteilhafterweise umfasst zumindest einer der Koppelelement-Sperrkreise eine Koppelelement-Spule und einen Koppelelement-Kondensator, die parallel geschaltet sind. Hierbei weist die Koppelelement-Spule bevorzugt eine Induktivität im Bereich von 38 bis 41 nH, besonders bevorzugt im Bereich von 39 bis 40 nH, insbesondere von 39 nH oder 40 nH auf. Der Koppelelement-Kondensator weist vorzugsweise eine Kapazität im Bereich von 6 bis 8 pF, insbesondere von 6,8 pF auf. Zumindest ein Koppelelement kann einen zweiten Koppelelement-Kondensator umfassen, der mit dem Koppelelement-Sperrkreis des Koppelelements in Reihe geschaltet ist. Der zweite Koppelelement-Kondensator weist bevorzugt eine Kapazität im Bereich von 20 bis 110 pF, besonders bevorzugt im Bereich von 33 bis 100 pF, insbesondere von 33 pF, 95 pF oder 100 pF auf. Zweckmäßigerweise ist der zweite Koppelelement-Kondensator ausgebildet, weist insbesondere eine geeignete Kapazität auf, um die Spulenanordnung auf die zweite Frequenz abzustimmen, insbesondere feinabzustimmen, und/oder einen Kurzschluss bei der zweiten Frequenz auszulösen. Ein Kapazitätswert des Koppelelement-Kondensators und des zweiten Koppelelement-Kondensators in der vorstehend genannten Größenordnung ist leicht realisierbar, was dazu beiträgt, dass sich die Spulenanordnung mit relativ geringem Aufwand abstimmen lässt. Die zuvor erwähnten Kapazitäts- und Induktivitätswerte sind insbesondere vorteilhaft bei einer Verwendung der Spulenanordnung als Teil eines MR-Systems, welches einen Magneten umfasst, der ausgebildet ist, um ein $B_0$ von 7T zu erzeugen.

Eine weitere Ausgestaltung der Erfindung zeichnet sich dadurch aus, dass sich die axialen Enden des stabförmigen Basiselements zumindest einer Dipolantenne jeweils unter Zwischenschaltung eines Anschlussstellen-Kondensators an das jeweilige Leiterbahnsegment der Dipolantenne anschließen. Der Anschlussstellen-Kondensator weist bevorzugt eine Kapazität im Bereich von 10 bis 40 pF, besonders bevorzugt im Bereich von 18 bis 32 pF, insbesondere von 18 pF oder 32 pF auf. Vorteilhaft ist der Anschlussstellen-Kondensator ausgebildet, weist insbesondere eine geeignete Kapazität auf, um die Spulenanordnung auf die zweite Frequenz abzustimmen, insbesondere feinabzustimmen. Die Anschlussstellen-Kondensatoren bilden somit einen weiteren Freiheitsgrad bei der Abstimmung, insbesondere wenn andere Kondensatoren, die bevorzugt ebenfalls zur Abstimmung vorgesehen sind, bereits durch Randbedingungen festgelegt sind.

Bevorzugt ist die Spulenanordnung insbesondere mittels der zweiten Verbindungselement-Kondensatoren und/oder der zweiten Koppelelement-Kondensatoren und/oder der Dipol-Kondensatoren und/oder der Anschlussstellen-Kondensatoren derart abgestimmt, dass jede Dipolantenne im elektrisch trennenden Zustand der Verbindungselemente und insbesondere der Koppelelemente ein hochfrequentes, elektromagnetisches Wechselfeld mit einer $^{1}$H-Kern-Resonanzfrequenz abstrahlen und/oder aufnehmen kann, und die sich im elektrisch verbindenden Zustand der Verbindungselemente und insbesondere der Koppelelemente ergebende Volumenspule und/oder jede Leiterschleife der sich im elektrisch verbindenden Zustand der Verbindungselemente und insbesondere der Koppelelemente ergebenden Leiterschleifenanordnung ein hochfrequentes, elektromagnetisches Wechselfeld mit einer X-Kern-Resonanzfrequenz abstrahlen und/oder aufnehmen kann.

In der nachstehenden Tabelle 1 sind vorteilhafte Kapazitäts- und Induktivitätswerte der elektrischen Bauteile einer Anschlusseinrichtung für eine $^{1}$H/$^{31}$P-Abstimmung bei $B_0$=7T aufgeführt. Die entsprechenden Kapazitäts- und Induktivitätswerte für eine $^{1}$H/$^{23}$Na-Abstimmung bei $B_0$=7T stehen jeweils in Klammern dahinter. Hierbei steht $^{1}$H/$^{31}$P-Abstimmung für eine Abstimmung auf die $^{1}$H-Kern-Resonanzfrequenz von 300 MHz und die $^{31}$P-Kern-Resonanzfrequenz

von 120 MHz als X-Kern-Resonanzfrequenz bei $B_0$=7T. $^1H/^{23}Na$-Abstimmung steht entsprechend für eine Abstimmung auf die $^1H$-Kern-Resonanzfrequenz von 300 MHz und die $^{23}Na$-Kern-Resonanzfrequenz von 78,82 MHz als X-Kern-Resonanzfrequenz bei $B_0$=7T.

Tabelle 1:

| Koppelelement-Spule Induktivität [nH] | Koppelelement-Kondensator Kapazität [pF] | Dipol-Kondensator Kapazität [pF] | Zweiter Koppelelement-Kondensator Kapazität [pF] |
|---|---|---|---|
| 40 | 6,8 | 0 | 33 oder 100 |
| (39) | (6,8) | (0) | (33 oder 95) |

Falls die Dipolantennenanordnung vier Dipolantennen umfasst, können die zweiten Koppelement-Kondensatoren der Koppelemente der Anschlusseinrichtungen zweier benachbarten Dipolantennen, insbesondere zweier in Umfangsrichtung der Volumenspule benachbarten Dipolantennen, jeweils eine gleiche Kapazität von insbesondere 33 pF aufweisen und die zweiten Koppelelement-Kondensatoren der beiden verbleibenden Dipolantennen ebenfalls eine gleiche Kapazität von bevorzugt 100 pF insbesondere für die $^1H/^{31}P$-Abstimmung oder 95 pF insbesondere für die $^1H/^{23}Na$-Abstimmung bei $B_0$=7T aufweisen. Bevorzugt handelt es sich bei den beiden Anschlusseinrichtungen, deren Koppelelement-Kondensatoren eine Kapazität von 33 pF aufweisen, um zwei Anschlusseinrichtungen, die nicht zum Einspeisen/Abgriff eines X-Kern-Signals verwendet werden.

In der nachstehenden Tabelle 2 sind vorteilhafte Kapazitäts- und Induktivitätswerte der elektrischen Bauteile eines Verbindungselements für die vorstehend beschriebene $^1H/^{31}P$-Abstimmung beziehungsweise in Klammern für die vorstehend beschriebene $^1H/^{23}Na$-Abstimmung aufgeführt.

Tabelle 2:

| Verbindungselement-Spule Induktivität [nH] | Verbindungselement-Kondensator Kapazität [pF] | Zweiter Verbindungselement-Kondensator Kapazität [pF] |
|---|---|---|
| 40 (39) | 6,8 (6,8) | 8,2 (40) |

Die Anschlussstellen-Kondensatoren können für die $^1H/^{31}P$-Abstimmung jeweils eine Kapazität von 18 pF und für die $^1H/^{23}Na$-Abstimmung jeweils eine Kapazität von 32 pF aufweisen.

Falls sich im elektrisch verbindenden Zustand der Verbindungselemente und insbesondere der Koppelemente eine Leiterschleifenanordnung ergibt, kann jede Leiterschleife der Leiterschleifenanordnung zumindest durch Teile zweier benachbarter Dipolantennen gebildet werden. Die Leiterschleifenanordnung umfasst bevorzugt mehrere Leiterschleifen, insbesondere drei Leiterschleifen. Zumindest ein Teil einer Dipolantenne, insbesondere, sofern vorhanden, zumindest deren stabförmiges Basiselement und bevorzugt, sofern vorhanden, zwei Anschlussstellen-Kondensatoren dieser, kann einen Teil zweier benachbarter Leiterschleifen bilden. In diesem Fall "teilen" sich zwei Leiterschleifen dann praktisch zumindest einen Teil einer Dipolantenne, insbesondere, sofern vorhanden, das stabförmige Basielement und die beiden Anschlussstellen-Kondensatoren dieser. Bildet zumindest ein Teil einer Dipolantenne einen Teil zweier benachbarter Leiterschleifen, sind bevorzugt die Kapazitätswerte der Verbindungselement-Kondensatoren und/oder der zweiten Verbindungselement-Kondensatoren und/oder der Koppelelement-Kondensatoren und/oder der zweiten Koppelelement-Kondensatoren und/oder der Dipol-Kondensatoren und/oder der Anschlussstellen-Kondensatoren und/oder weiterer Kondensatoren innerhalb der Leiterschleifenanordnung derart gewählt, dass benachbarte Leiterschleifen bevorzugt kapazitiv entkoppelt sind. Die Bestimmung der zur Entkopplung benachbarter Leiterschleifen benötigten Kapazitätswerte wird von A.L. Perrier, D. Grenier, N. Ravel, P. Litaudon und O. Beuf in dem Aufsatz "Capacitive approach to restore decoupling between channels for four-element MR coil array", ELECTRONICS LETTERS, 20. Juni 2013, Vol. 49, Nr. 13 beschrieben.

Es versteht sich, dass die erfindungsgemäße Spulenanordnung zur Verwendung als Sende- und/oder Empfangsspule in einem UHF-MRT-System geeignet sein kann.

Die zuvor erwähnte Aufgabe wird erfindungsgemäß auch durch ein MR-System, insbesondere MRT-, bevorzugt Hochfeld-/Ultrahochfeld-MRT-, und/oder MRS-, bevorzugt Hochfeld-/Ultrahochfeld-MRS-System mit der zuvor beschriebenen erfindungsgemäßen Spulenanordnung gelöst.

Das MR-System umfasst bevorzugt einen insbesondere supraleitenden Magneten, der ausgebildet ist, um ein statisches, bevorzugt homogenes Magnetfeld $B_0$ zu erzeugen, wobei $B_0$ vorzugsweise 7T beträgt. Die Spulenanordnung kann sich innerhalb einer zentralen Öffnung des Magneten befinden. Die Spulenanordnung kann einen Aufnahmeraum umgeben, in dem während einer MR-Untersuchung ein zu untersuchendes Medium angeordnet ist oder angeordnet werden kann.

Des Weiteren betrifft die Erfindung eine Verwendung der zuvor beschriebenen erfindungsgemäßen Spulenanordnung als Hochfrequenz-Sende- und/oder Empfangsspule bei der Magnetresonanztomographie, insbesondere Hochfeld-/Ultrahochfeld-Magnetresonanztomographie und/oder Magnetresonanzspektroskopie, insbesondere Hochfeld-/Ultrahochfeld-Magnetresonanzspektroskopie.

Weitere Merkmale und Vorteile der vorliegenden Erfindung werden anhand der nachfolgenden Beschreibung einer Ausführungsform einer Spulenanordnung gemäß der vorliegenden Erfindung unter Bezugnahme auf die beiliegende Zeichnung deutlich. Darin ist:

Figur 1 eine schematische perspektivische Ansicht einer erfindungsgemäßen Spulenanordnung gemäß einer Ausführungsform der vorliegenden Erfindung;

Figur 2 eine Art Explosionsansicht der Spulenanordnung der Figur 1, in der die Dipolantennen separiert dargestellt sind;

Figur 3 ein Schaltbild der Spulenanordnung der Figur 1;

Figur 4 ein Frequenzspektrum des Eingangsreflexionsfaktors, das die beiden Resonanzfrequenzen veranschaulicht, auf die die erfindungsgemäße Spulenanordnung abgestimmt ist;

Figur 5 eine grafische Darstellung der sicheren Übertragungseffizienz der erfindungsgemäßen Spulenanordnung im Vergleich mit der sicheren Übertragungseffizienz einer einfach abgestimmten 8-Kanal-Schleifenanordnung bei der $^{1}$H-Kern-Resonanzfrequenz von 300 MHz; und

Figur 6 eine grafische Darstellung der sicheren Übertragungseffizienz der erfindungsgemäßen Spulenanordnung im Vergleich mit der sicheren Übertragungseffizienz einer einfach abgestimmten Birdcage-Spule bei der $^{31}$P-Kern-Resonanzfrequenz von 120MHz.

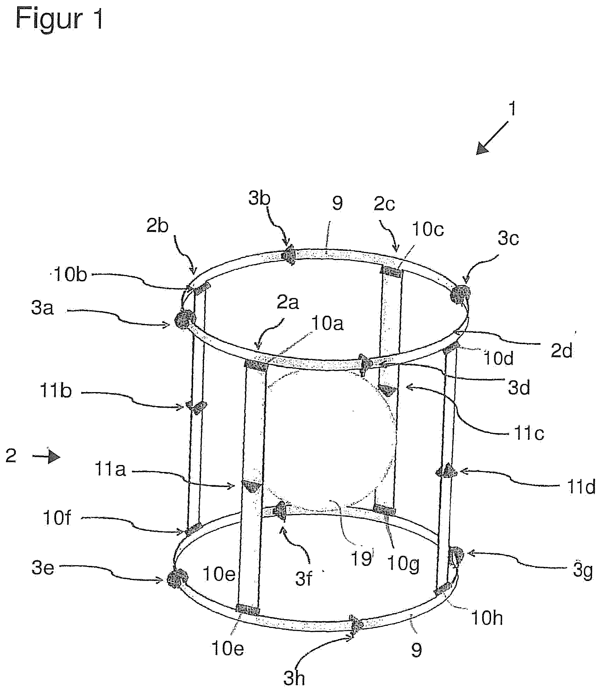

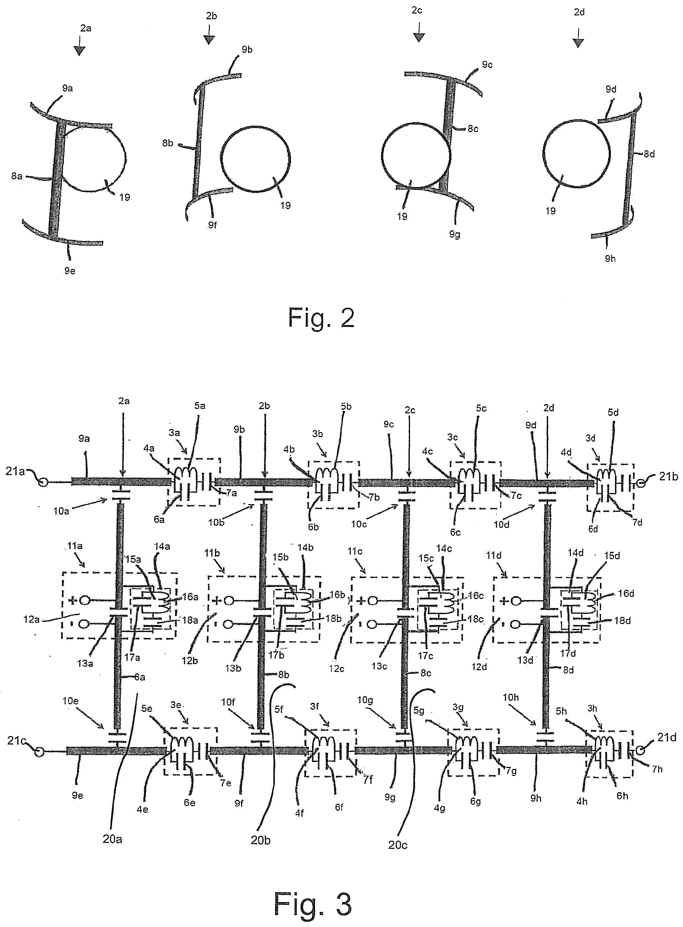

Die Figur 1 zeigt eine schematische Ansicht einer Spulenanordnung 1 gemäß einer Ausführungsform der vorliegenden Erfindung. Die Spulenanordnung 1 umfasst eine Dipolantennenanordnung 2 mit vier Dipolantennen 2a-d, die über acht Verbindungselemente 3a-h miteinander verbunden sind. Figur 2 zeigt eine Art Explosionsansicht der Spulenanordnung 1, in der die Dipolantennen 2a-d separat dargestellt sind. Die Verbindungselemente 3a-h sind ausgebildet, um von einem elektrisch verbindenden in einen elektrisch trennenden Zustand überführt zu werden, und umgekehrt. Die Anordnung ist erkennbar derart getroffen, dass die Dipolantennen 2a-d im elektrisch verbindenden Zustand der Verbindungselemente 3a-h eine zylindrische Volumenspule bilden.

Die Verbindungselemente 3a-h umfassen jeweils einen Verbindungselement-Sperrkreis 4a-h, der automatisch sperrt, wenn eine hochfrequente Wechselspannung mit einer der Sperrfrequenz der Verbindungselement-Sperrkreise 4a-h entsprechenden Frequenz an die Spulenanordnung 1 angelegt und/oder darin induziert wird. Dieser ist in dem in Figur 3 gezeigten Schaltplan der Spulenanordnung 1 zu erkennen. Die Verbindungselement-Sperrkreise 4a-h umfassen jeweils eine Verbindungselement-Spule 5a-h und einen Verbindungselement-Kondensator 6a-h, die parallel geschaltet sind. Außerdem umfasst jedes Verbindungselement 3a-h einen zweiten Verbindungselement-Kondensator 7a-h, der mit dem Verbindungselement-Sperrkreis 4a-h in Reihe geschaltet ist.

Die Dipolantennen 2a-d weisen jeweils ein stabförmiges Basiselement 8a-d auf, an dessen axial gegenüberliegenden Enden sich jeweils ein ringsegmentartiges Leiterbahnsegment 9a-h anschließt. Hierbei schließen sich die axialen Enden des stabförmigen Basiselements 8a-d unter Zwischenschaltung eines Anschlussstellen-Kondensators 10a-h mittig an das jeweilige Leiterbahnsegment 9a-h an. Die stabförmigen Basiselemente 8a-d der Dipolantennen 2a-d sind parallel zueinander und in Umfangsrichtung der Volumenspule gleichmäßig voneinander beabstandet angeordnet. Die Leiterbahnsegmente 9a-h sind gleich groß, weisen genauer gesagt die gleiche Bogenlänge auf. Außerdem sind die ringsegmentartigen Leiterbahnsegmente 9a-h über die Verbindungselemente 3a-h unter Bildung zweier ringförmig geschlossenen Leiterbahnen 9 miteinander verbunden. Somit sind in jeder Leiterbahn 9 vier Verbindungselemente 3a-h angeordnet.

Die stabförmigen Basiselemente 8a-d der Dipolantennen 2a-d sind jeweils mittig unter Bildung zweier Pole der jeweiligen Dipolantenne 2a-d aufgetrennt. Darüber hinaus sind die stabförmigen Basiselemente 8a-d jeweils in ihrem mittleren Abschnitt mit einer Anschlusseinrichtung 11a-d zur Verbindung mit einer nicht dargestellten Wechselspannungsversorgungs- und aufnahmeeinrichtung versehen. Die Anschlusseinrichtung 11a-d umfasst mit den beiden Polen der Dipolantenne 2a-d verbundene, elektrische Anschlusselemente 12a-d. Außerdem weist die Anschlusseinrichtung 11a-d einen Dipol-Kondensator 13a-d auf, der in der Mitte der Dipolantenne 2a-d zwischen den beiden Polen vorgesehen ist. Darüber hinaus umfasst die Anschlusseinrichtung 11a-d ein parallel zu dem Dipol-Kondensator 13a-d geschaltetes Koppelelement 14a-d, das mit den beiden Polen der Dipolantenne 2a-d elektrisch verbunden ist und diese überbrückt. Die insgesamt vier Koppelelemente 14a-d sind jeweils ausgebildet, um von einem elektrisch verbindenden in einen elektrisch trennenden Zustand überführt zu werden, und umgekehrt.

Hierzu umfassen die Koppelelemente 14a-d jeweils einen Koppelelement-Sperrkreis 15a-d, der eine Koppelelement-Spule 16a-d und einen Koppelelement-Kondensator 17a-d, die parallel geschaltet sind, aufweist. Die Koppelelement-Sperrkreise 15a-d sperren automatisch, wenn eine hochfrequente Wechselspannung mit einer der Sperrfrequenz der Koppelelement-Sperrkreise 15a-d entsprechenden Frequenz an die Spulenanordnung 1 angelegt und/oder darin induziert wird. Des Weiteren umfassen die Koppelelemente 14a-d jeweils einen zweiten Koppelelement-Kondensator 18a-d, der mit dem Koppelelement-Sperrkreis 15a-d in Reihe geschaltet ist.

Die sich im elektrisch verbindenden Zustand der Verbindungselemente 3a-h ergebende zylindrische Volumenspule ist eine Volumenspule nach Art einer Alderman-Grant-Spule mit zwei Endringen, hier die ringförmig geschlossenen Leiterbahnen 9, und vier Stegen, hier die stabförmigen Basiselemente 8a-d der Dipolantennen 2a-d. Die Spulenanordnung 1 ist vorliegend 28 cm lang und weist vorliegend einen Durchmesser von 26 cm auf. In den Figuren 1 und 2 ist ein kugelförmiges Phantom 19 innerhalb der Spulenanordnung 1 dargestellt. Dieses ist stellvertretend für ein zu untersuchendes Körperteil, insbesondere einen zu untersuchenden menschlichen oder tierischen Kopf.

In der folgenden Tabelle sind die elektrischen Bauteile der vier Anschlusseinrichtungen 11a-d nochmals kurz beschrieben und deren Werte aufgeführt.

| Anschluss einrichtung | Koppelelement-Spule 16a-d | | Koppelelement-Kondensator 17a-d | | Dipol-Kondensator 13a-d | | Zweiter Koppelelement-Kondensator 18a-d | |
|---|---|---|---|---|---|---|---|---|
| | Beschreibung | Wert (nH) | Beschreibung | Wert (pF) | Beschreibung | Wert (pF) | Beschreibung | Wert (pF) |
| 11a | Spule 16a für $^1H$-Koppelelement-Sperrkreis 15a | 40 | Kondensator 17a für $^1H$-Koppelelement-Sperrkreis 15a | 6,8 | Kondensator 13a für dipole Anpassung | 0 | Kondensator 18a für Abstimmung, die Kurzschluss bei der X-Kern-Frequenz auslöst | 33 |
| 11b | Spule 16b für $^1H$-Koppelelement-Sperrkreis 15b | 40 | Kondensator 17b für $^1H$-Koppelelement-Sperrkreis 15b | 6,8 | Kondensator 13b für dipole Anpassung | 0 | Kondensator 18b für Abstimmung, die Kurzschluss bei der X-Kern-Frequenz auslöst | 33 |
| 11c | Spule 16c für $^1H$-Koppelelement-Sperrkreis 15c | 40 | Kondensator 17c für $^1H$-Koppelelement-Sperrkreis 15c | 6,8 | Kondensator 13c für dipole Anpassung | 0 | Kondensator 18c für X-Kem-Anpassung | 100 |
| 11d | Spule 16d für $^1H$-Koppelelement-Sperrkreis 15d | 40 | Kondensator 17d für $^1H$-Koppelelement-Sperrkreis 15d | 6,8 | Kondensator 13d für dipole Anpassung | 0 | Kondensator 18d für X-Kem-Anpassung | 100 |

In der folgenden Tabelle sind die elektrischen Bauteile der acht Verbindungselemente 3a-h nochmals kurz beschrieben und deren Werte aufgeführt.

| Verbindungselemente | Verbindungselement-Spule 5a-h | | Verbindungselement-Kondensator 6a-h | | Zweiter Verbindungselement-Kondensator 7a-h | |
|---|---|---|---|---|---|---|
| | Beschreibung | Wert (nH) | Beschreibung | Wert (pF) | Beschreibung | Wert (pF) |
| 3a | Spule 5a für $^{1}$H-Verbindungs-element-Sperrkreis 4a | 40 | Kondensator 6a für $^{1}$H-Verbin-dungselement-Sperrkreis 4a | 6,8 | Abstimmung für X-Kerne | 8,2 |
| 3b | Spule 5b für $^{1}$H-Verbindungs-element-Sperrkreis 4b | 40 | Kondensator 6b für $^{1}$H-Verbin-dungselement-Sperrkreis 4b | 6,8 | Abstimmung für X-Kerne | 8,2 |
| 3c | Spule 5c für $^{1}$H-Verbindungsele-ment-Sperrkreis 4c | 40 | Kondensator 6c für $^{1}$H-Verbin-dungselement-Sperrkreis 4c | 6,8 | Abstimmung für X-Kerne | 8,2 |
| 3d | Spule 5d für $^{1}$H-Verbindungs-element-Sperrkreis 4d | 40 | Kondensator 6d für $^{1}$H-Verbin-dungselement-Sperrkreis 4d | 6,8 | Abstimmung für X-Kerne | 8,2 |
| 3e | Spule 5e für $^{1}$H-Verbindungs-element-Sperrkreis 4e | 40 | Kondensator 6e für $^{1}$H-Verbin-dungselement-Sperrkreis 4e | 6,8 | Abstimmung für X-Kerne | 8,2 |
| 3f | Spule 5f für $^{1}$H-Verbindungsele-ment-Sperrkreis 4f | 40 | Kondensator 6f für $^{1}$H-Verbin-dungselement-Sperrkreis 4f | 6,8 | Abstimmung für X-Kerne | 8,2 |
| 3g | Spule 5g für $^{1}$H-Verbindungs-element-Sperrkreis 4g | 40 | Kondensator 6g für $^{1}$H-Verbin-dungselement-Sperrkreis 4g | 6,8 | Abstimmung für X-Kerne | 8,2 |
| 3h | Spule 5h für $^{1}$H-Verbindungs-element-Sperrkreis 4h | 40 | Kondensator 6h für $^{1}$H-Verbin-dungselement-Sperrkreis 4h | 6,8 | Abstimmung für X-Kerne | 8,2 |

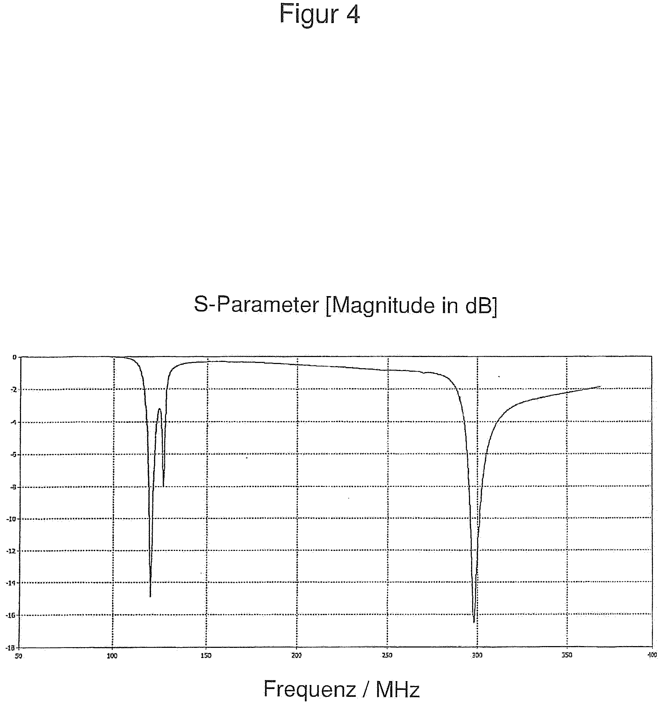

In Figur 4 ist ein Frequenzspektrum des Eingangsreflexionsfaktors der erfindungsgemäßen Spulenanordnung 1 dargestellt, die durch die in den vorstehenden Tabellen aufgeführten Kapazitäts- und Induktivitätswerte abgestimmt ist. Die Anschlussstellen-Kondensatoren 10a-h und insbesondere auch die Verbindungselement-Spulen 5a-h und die Koppelelement-Spulen 16a-d tragen ebenfalls zur Abstimmung bei. Vorliegend beträgt die Kapazität der Anschlussstellen-Kondensatoren 10a-h jeweils 18 pF. Es ist zu erkennen, dass das Spulensystem bei $B_0$=7 T doppelt abgestimmt ist, nämlich auf die $^1$H-Kern-Resonanzfrequenz von 300 MHz und auf eine X-Kern-Resonanzfrequenz, nämlich die $^{31}$P-Kern-Resonanzfrequenz von 120 MHz.

Bei einer Verwendung der auf die zuvor beschriebene Weise doppelt abgestimmten, erfindungsgemäßen Spulenanordnung 1 in einem MRT-System mit $B_0$=7 T werden in einem ersten Betriebsmodus des MRT-Systems die Dipolantennen 2a-d über die elektrischen Anschlusselemente 12a-d ihrer Anschlusseinrichtungen 11a-d jeweils mit einer hochfrequenten Wechselspannung gespeist. Hierbei kann die Phasenbeziehung zwischen den an den vier Anschlusseinrichtungen 11a-d angelegten Speisesignalen beispielsweise 0 -90 -180 -270 Grad betragen. Die Frequenz der hochfrequenten Wechselspannung entspricht einer gemeinsamen Sperrfrequenz der Verbindungselement-Sperrkreise 4a-h und der Koppelelement-Sperrkreise 15a-d. Hierbei entspricht die gemeinsame Sperrfrequenz der $^1$H-Kern-Resonanzfrequenz. Daraufhin sperren die Verbindungselement-Sperrkreise 4a-h und die Koppelelement-Sperrkreise 15a-d automatisch, wodurch die Verbindungselemente 3a-h und die Koppelelemente 14a-d in ihren elektrisch trennenden Zustand überführt werden. Im elektrisch trennenden Zustand strahlt jede Dipolantenne 2a-d ein hochfrequentes, elektromagnetisches Wechselfeld mit der $^1$H-Kern-Resonanzfrequenz von 300 MHz ab. Später wird eine aufgrund angeregter $^1$H-Kerne in der Spulenanordnung 1 induzierte hochfrequente elektrische Wechselspannung an den Anschlusseinrichtungen 11a-d abgegriffen.

In einem zweiten Betriebsmodus des MRT-Systems wird die Spulenanordnung 1 über die elektrischen Anschlusselemente 12c und 12d der beiden in Umfangsrichtung der Volumenspule benachbarten Anschlusseinrichtungen 11c und 11d in einem Quadraturbetrieb mit einer hochfrequenten Wechselspannung gespeist, wobei die Anschlusseinrichtungen 11c und 11d mit um 90 Grad phasenverschobenen Signalen angesteuert werden. Hierbei unterscheidet sich die Frequenz der hochfrequenten Wechselspannung von der gemeinsamen Sperrfrequenz. Somit befinden sich die Verbindungselemente 3a-h und die Koppelelemente 14a-d in ihrem elektrisch verbindenden Zustand. Daraufhin strahlt die sich im elektrisch verbindenden Zustand ergebende Volumenspule ein hochfrequentes, elektromagnetisches Wechselfeld mit der $^{31}$P-Kern-Resonanzfrequenz von 120 MHz ab. Später wird eine aufgrund angeregter $^{31}$P-Kerne in der Spulenanordnung 1 induzierte elektrische Wechselspannung an den Anschlusseinrichtungen 11c und 11d abgegriffen. Die zuvor beschriebene erfindungsgemäße Spulenanordnung 1 wurde als Hochfrequenz-Spule in einem MRT-System verwendet.

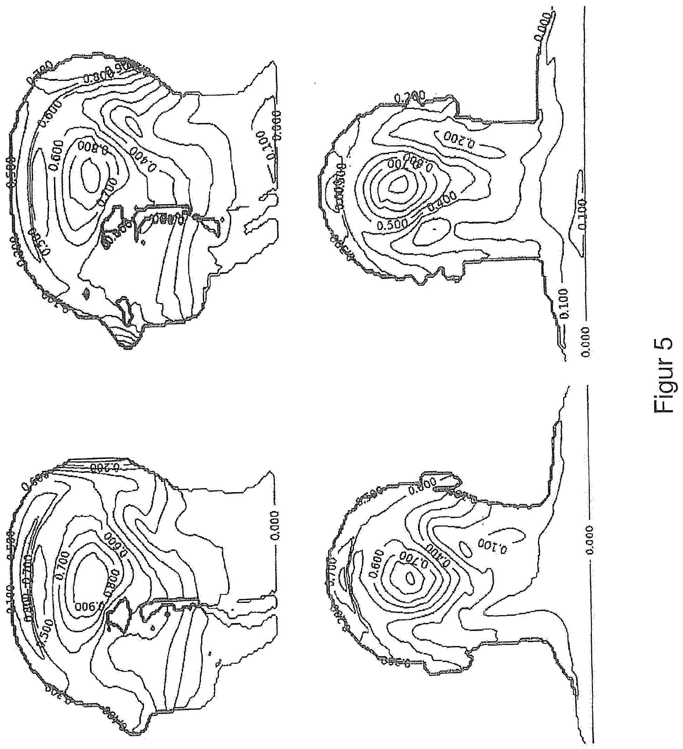

Es wurde zunächst die elektromagnetische Feldverteilung im Kopfe eines Probanden simuliert, für den Fall, dass die $^1$H-Kerne angeregt und anschließend detektiert wurden. Mit $B_0$=7 T betrug die $^1$H-Kern-Resonanzfrequenz beziehungsweise $^1$H-Kern-Lamorfrequenz 300 MHz. Die gleiche MRT-Untersuchung wurde nochmals mit einer Vergleichsanordnung durchgeführt, bei der als Hochfrequenz-Spule eine 8-Kanal-Spulenanordnung verwendet wurde, die lediglich auf die $^1$H-Kern-Resonanzfrequenz abgestimmt war. Konkret wurden bei der Vergleichsanordnung acht Leiterschleifen um den Kopf des Probanden herumgelegt. Die einfach abgestimmte 8-KanalAnordnung lieferte quasi eine optimale Vergleichsmessung für eine $^1$H-Untersuchung. Die resultierenden MRT-Schnittbilder sowohl bei Verwendung der erfindungsgemäßen Spulenanordnung 1 als auch bei Verwendung der Vergleichsanordnung sind in Figur 5 in Form einer Matrix dargestellt. In der linken Spalte befinden sich die Schnittbilder für die Vergleichsanordnung und in der rechten Spalte sind die Schnittbilder für die erfindungsgemäße Spulenanordnung 1 aufgeführt. In der oberen Zeile sind die Schnittbilder in der Sagittalebene und in der unteren Zeile die Schnittbilder in der Frontal- oder Coronalebene gezeigt. Konkret ist ein Vergleich der sicheren Übertragungseffizienz beider MRT-Untersuchungen dargestellt. Hierbei ist die sichere Übertragungseffizienz als Amplitude des zirkular polarisierten hochfrequenten Anregungsfeldes geteilt durch die maximale spezifische Absorptionsrate $B_1^+/\mathrm{sqrt(SAR)}$ definiert. Der Vergleich zeigt, dass sich die sichere Übertragungseffizienz bei Verwendung der erfindungsgemäßen Spulenanordnung 1 mit einem Mittelwert von 0,48 $\mu T/\sqrt{W}$ über das Gehrin des Probanden und die quasi als optimal anzusehende sichere Übertragungseffizienz bei Verwendung der Vergleichsanordnung mit einem Mittelwert von 0,51 $\mu T/\sqrt{W}$ ähneln, obwohl es sich bei der erfindungsgemäßen Spulenanordnung 1 um eine doppelt abgestimmte beziehungsweise doppelt resonante Spulenanordnung handelt. Dies deutet auf einen nur geringen Verlust hin.

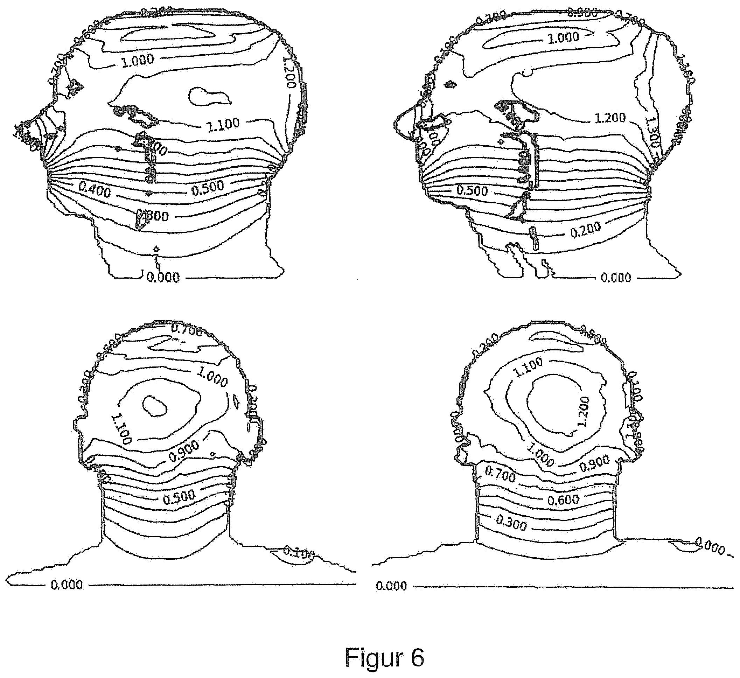

Außerdem wurde eine Simulation der Feldverteilung im Kopfe eines Probanden durchgeführt, bei der die $^{31}$P-Kerne angeregt und anschließend detektiert wurden. Mit $B_0$=7 T betrug die $^{31}$P-Kern-Resonanzfrequenz beziehungsweise $^{31}$P-Kern-Lamorfrequenz 120 MHz. Die gleiche MRT-Untersuchung wurde nochmals mit einer Vergleichsanordnung durchgeführt, bei der als Hochfrequenz-Spule eine Birdcage-Spule verwendet wurde, die lediglich auf die $^{31}$P-Kern-Resonanzfrequenz abgestimmt war. Die einfach abgestimmte Birdcage-Spule lieferte quasi eine optimale Vergleichsmessung für eine $^{31}$P-Untersuchung. Die resultierenden MRT-Schnittbilder sowohl bei **Verwendung** der erfindungsgemäßen Spulenanordnung 1 als auch bei Verwendung der Vergleichsanordnung sind in Figur 6 in Form einer Matrix dargestellt. In der rechten Spalte befinden sich die Schnittbilder für die erfindungsgemäße Spulenanordnung 1 und in der linken Spalte sind die Schnittbilder für die Vergleichsanordnung aufgeführt. In der oberen Zeile sind die Schnittbilder in der Sagittalebene und in der unteren Zeile die Schnittbilder in der Frontal- oder Coronalebene gezeigt. Der dargestellte Vergleich der sicheren Übertragungseffizienz beider MRT-Untersuchungen zeigt, dass sich die sichere Übertragungseffizienz bei Verwendung der erfindungsgemäßen Spulenanordnung 1 mit einem Mittelwert von 1,09 $\mu T/\sqrt{W}$ über das Gehirn des Probanden und die quasi als optimal anzusehende sichere Übertragungseffizienz bei Verwendung der Vergleichsanordnung mit einem Mittelwert von 1,00 $\mu T/\sqrt{W}$ ähneln, obwohl es sich bei der erfindungsgemäßen Spulenanordnung 1 um eine doppelt abgestimmte beziehungsweise doppelt resonante Spulenanordnung handelt. Dies deutet auf einen nur geringen Verlust hin.

Bei der zuvor beschriebenen Ausführungsform einer erfindungsgemäßen Spulenanordnung 1 ergibt sich im elektrisch verbindenden Zustand der Verbindungselemente 3a-h und der Koppelelemente 14a-d eine Volumenspule. In einer alternativen Ausführungsform kann sich anstatt einer Volumenspule eine flache Leiterschleifenanordnung ergeben, die beispielsweise eine flache Struktur ähnlich der Darstellung von Figur 3 aufweisen kann, jedoch ohne die beiden Verbindungselemente 3d und 3h. Die Leiterschleifenanordnung umfasst in diesem Fall drei Leiterschleifen 20a-c, die jeweils zumindest durch Teile zweier benachbarter Dipolantennen 2a-d gebildet werden. Im vorliegenden Beispiel bildet ein Teil der Dipolantenne 2b sowohl einen Teil der Leiterschleife 20a als auch einen Teil der Leiterschleife 20b. Außerdem bildet ein Teil der Dipolantenne 2c sowohl einen Teil der Leiterschleife 20b als auch einen Teil der Leiterschleife 20c. Genauer gesagt wird die Leiterschleife 20a durch das stabförmige Basiselement 8a, die beiden Anschlussstellen-Kondensatoren 10a und 10e und jeweils eine Hälfte der Leiterbahnsegmente 9a und 9e der Dipolantenne 2a sowie das stabförmige Basiselement 8b, die beiden Anschlussstellen-Kondensatoren 10b und 10f und jeweils eine Hälfte der Leiterbahnsegmente 9b und 9f der Dipolantenne 2b gebildet. Die Leiterschleife 20b wird durch das stabförmige Basiselement 8b, die beiden Anschlussstellen-Kondensatoren 10b und 10f und jeweils eine Hälfte der Leiterbahnsegmente 9b und 9f der Dipolantenne 2b sowie das stabförmige Basiselement 8c, die beiden Anschlussstellen-Kondensatoren 10c und 10g und jeweils eine Hälfte der Leiterbahnsegmente 9c und 9g der Dipolantenne 2c gebildet. Zuletzt wird die Leiterschleife 20c durch das stabförmige Basiselement 8c, die beiden Anschlussstellen-Kondensatoren 10c und 10g und jeweils eine Hälfte der Leiterbahnsegmente 9c und 9g der Dipolantenne 2c sowie das stabförmige Basiselement 8d, die beiden Anschlussstellen-Kondensatoren 10d und 10h und jeweils eine Hälfte der Leiterbahnsegmente 9d und 9h der Dipolantenne 2d gebildet. Somit "teilen" sich die Leiterschleifen 20a und 20b das stabförmige Basiselement 8b und die beiden Anschlussstellen-Kondensatoren 10b und 10f der Dipolantenne 2b. Die Leiterschleifen 20b und 20c "teilen" sich das stabförmige Basiselement 8c und die beiden Anschlussstellen-Kondensatoren 10c und 10g der Dipolantenne 2c. Die Kapazitätswerte aller Kondensatoren innerhalb der Leiterschleifenanordnung sind derart gewählt, dass benachbarte Leiterschleifen 20a-c bevorzugt kapazitiv entkoppelt sind.

Im Falle einer flachen Leiterschleifenanordnung sind die beiden Leiterbahnen 2 bevorzugt geradlinig und nicht ringförmig. Eine Einspeisung und/oder ein Abgriff von $^{1}$H-Kern- und/oder X-Kern-Signalen kann bei einer flachen Leiterschleifenanordnung alternativ zu den Anschlusseinrichtungen 11a-d über die freien Enden 21a-d der Leiterbahnen erfolgen. Im Falle einer flachen Leiterschleifenanordnung erfolgt das Abstrahlen und/oder die Aufnahme des hochfrequenten, elektromagnetischen Wechselfeldes mit der X-Kern-Resonanzfrequenz über die einzelnen Leiterschleifen 20a-c. Das Abstrahlen und/oder die Aufnahme des hochfrequenten, elektromagnetischen Wechselfeldes mit der $^{1}$H-Kern-Resonanzfrequenz erfolgt über die einzelnen Dipolantennen 2a-d.

Bezugszeichenliste

| | |
|---|---|
| 1 | Spulenanordnung |
| 2 | Dipolantennenanordnung |
| 2a-d | Dipolantennen |
| 3a-h | Verbindungselemente |
| 4a-h | Verbindungselement-Sperrkreis |
| 5a-h | Verbindungselement-Spule |
| 6a-h | Verbindungselement-Kondensator |
| 7a-h | zweiter Verbindungselement-Kondensator |
| 8a-d | stabförmiges Basiselement |
| 9 | Leiterbahn |
| 9a-h | Leiterbahnsegment |

| | |
|---|---|
| 10a-h | Anschlussstellen-Kondensator |
| 11a-d | Anschlusseinrichtung |
| 12a-d | Anschlusselement |
| 13a-d | Dipol-Kondensator |
| 14a-d | Koppelelement |
| 15a-d | Koppelelement-Sperrkreis |
| 16a-d | Koppelelement-Spule |
| 17a-d | Koppelelement-Kondensator |
| 18a-d | zweiter Koppelelement-Kondensator |
| 19 | Phantom |
| 20a-c | Leiterschleife |
| 21 | freies Ende |

**Patentansprüche**

1. Spulenanordnung (1) zur Verwendung als Sende- und/oder Empfangsspule in einem MR-System, insbesondere MRT- und/oder MRS-System, die eine Dipolantennenanordnung (2) mit mehreren über Verbindungselemente (3a-h) miteinander verbundenen Dipolantennen (2a-d) umfasst, wobei die Verbindungselemente (3a-h) ausgebildet sind, um von einem elektrisch verbindenden in einen elektrisch trennenden Zustand überführt zu werden, und umgekehrt, und die Anordnung derart getroffen ist, dass die Dipolantennen (2a-d) im elektrisch verbindenden Zustand der Verbindungselemente (3a-h) zumindest einen Teil einer bevorzugt zylindrischen Volumenspule und/oder eine mindestens eine Leiterschleife (20a-c) umfassende, insbesondere flache Leiterschleifenanordnung der Spulenanordnung (1) bilden, wobei die Verbindungselemente (3a-h) Verbindungselement-Sperrkreise (4a-h) umfassen, die automatisch sperren, wenn eine hochfrequente Wechselspannung mit einer der Sperrfrequenz der Verbindungselement-Sperrkreise (4a-h) entsprechenden Frequenz an die Spulenanordnung (1) angelegt wird, wobei die Spulenanordnung (1) derart ausgestaltet ist, dass die Dipolantennen (2a-d) im elektrisch trennenden Zustand der Verbindungselemente (3a-h) ein hochfrequentes, elektromagnetisches Wechselfeld mit einer ersten, der Sperrfrequenz entsprechenden Frequenz abstrahlen und/oder aufnehmen, und dass die sich im elektrisch verbindenden Zustand der Verbindungselemente (3a-h) ergebende Volumenspule und/oder jede Leiterschleife (20a-c) der sich im elektrisch verbindenden Zustand der Verbindungselemente (3a-h) ergebenden Leiterschleifenanordnung ein hochfrequentes, elektromagnetisches Wechselfeld mit einer zweiten von der ersten verschiedenen Frequenz abstrahlt und/oder aufnimmt.

2. Spulenanordnung (1) nach Anspruch 1, **dadurch gekennzeichnet, dass** die erste Frequenz eine $^{1}$H-Kern-Resonanzfrequenz und die zweite Frequenz eine X-Kern-Resonanzfrequenz, insbesondere eine $^{31}$P-Kern- oder $^{23}$Na-Kern-Resonanzfrequenz, ist.

3. Spulenanordnung (1) nach Anspruch 1 oder 2, **dadurch gekennzeichnet, dass** die Dipolantennen (2a-d) jeweils ein stabförmiges Basiselement (8a-d) umfassen, an dessen axial gegenüberliegenden Enden sich jeweils ein insbesondere ringsegmentartiges Leiterbahnsegment (9a-h) anschließt, wobei sich die axialen Enden des stabförmigen Basiselements (8a-d) insbesondere mittig an das jeweilige Leiterbahnsegment (9a-h) anschließen, und wobei die Leiterbahnsegmente (9a-h) der Dipolantennen (2a-d) bevorzugt gleich groß sind, insbesondere die gleiche Bogenlänge aufweisen, insbesondere, wobei die stabförmigen Basiselemente (8a-d) der Dipolantennen (2a-d) zumindest im Wesentlichen parallel zueinander angeordnet sind und/oder die stabförmigen Basiselemente (8a-d) der Dipolantennen (2a-d) in Umfangsrichtung der Volumenspule oder in Längsrichtung der Leiterschleifenanordnung gleichmäßig voneinander beabstandet angeordnet sind und/oder die Länge der stabförmigen Basiselemente (8a-d) der Dipolantennen (2a-d), die Höhe der Volumenspule oder die Breite der Leiterschleifenanordnung im Bereich von 25 bis 30 cm liegt, insbesondere 25 cm oder 28 cm beträgt, und/oder die Leiterbahnsegmente (9a-h) der Dipolantennen (2a-d) über die Verbindungselemente (3a-h) unter Bildung zweier Leiterbahnen (9), insbesondere zweier ringförmig geschlossener Leiterbahnen (9), miteinander verbunden sind, und insbesondere die Anzahl an Verbindungselementen (3a-h) pro Leiterbahn (9) um eins geringer als die Anzahl an Dipolantennen (2a-d) ist oder der Anzahl an Dipolantennen (2a-d) entspricht, und/oder der Durchmesser der ringförmig geschlossenen Leiterbahnen (9) oder der Volumenspule im Bereich von 25 bis 30 cm liegt, insbesondere 26 cm beträgt.

4. Spulenanordnung (1) nach einem der vorhergehenden Ansprüche, **dadurch gekennzeichnet, dass** zumindest einer der Verbindungselement-Sperrkreise (4a-h) eine Verbindungselement-Spule (5a-h) und einen Verbindungselement-Kondensator (6a-h) umfasst, die parallel geschaltet sind, wobei die Verbindungselement-Spule (5a-h) bevorzugt eine Induktivität im Bereich von 38 bis 41 nH, besonders bevorzugt im Bereich von 39 bis 40 nH, insbesondere von 39 nH oder 40 nH aufweist und/oder der Verbindungselement-Kondensator (6a-h) bevorzugt eine Kapazität im Bereich von 6 bis 8 pF, insbesondere von 6,8 pF aufweist.

5. Spulenanordnung (1) nach Anspruch 4, **dadurch gekennzeichnet, dass** zumindest ein Verbindungselement (3a-h) einen zweiten Verbindungselement-Kondensator (7a-h) umfasst, der mit dem Verbindungselement-Sperrkreis (4a-h) in Reihe geschaltet ist, wobei der zweite Verbindungselement-Kondensator (7a-h) bevorzugt eine Kapazität im Bereich von 5 bis 50 pF, besonders bevorzugt im Bereich von 8,2 bis 40 pF, insbesondere von 8,2 pF oder 40 pF aufweist.

6. Spulenanordnung (1) nach Anspruch 5, **dadurch gekennzeichnet, dass** der zweite Verbindungselement-Kondensator (7a-h) ausgebildet ist, insbesondere eine geeignete Kapazität aufweist, um die Spulenanordnung (1) auf die zweite Frequenz abzustimmen, insbesondere feinabzustimmen.

7. Spulenanordnung (1) nach Anspruch 3 oder einem der Ansprüche 4 bis 6, sofern auf Anspruch 3 rückbezogen, **dadurch gekennzeichnet, dass** die stabförmigen Basiselemente (8a-d) der Dipolantennen (2a-d) jeweils bevorzugt mittig unter Bildung zweier Pole der jeweiligen Dipolantenne (2a-d) aufgetrennt sind.

8. Spulenanordnung (1) nach Anspruch 7, **dadurch gekennzeichnet, dass** die stabförmigen Basiselemente (8a-d) der Dipolantennen (2a-d) jeweils mit einer Anschlusseinrichtung (11a-d) zur Verbindung mit einer Wechselspannungsversorgungs- und/oder aufnahmeeinrichtung versehen sind, die mit den beiden Polen der Dipolantenne (2a-d) verbundene elektrische Anschlusselemente (12a-d) umfasst.

9. Spulenanordnung (1) nach Anspruch 2 und Anspruch 8, **dadurch gekennzeichnet, dass** die Spulenanordnung (1) derart ausgestaltet ist, dass eine Einspeisung und/oder ein Abgriff von $^{1}$H-Kern-Signalen und/oder X-Kern-Signalen über die elektrischen Anschlusselemente (12a-d) zumindest einer Anschlusseinrichtung (11a-d), insbesondere aller Anschlusseinrichtungen (11a-d), erfolgen kann, bevorzugt, wobei die Spulenanordnung (1) derart ausgestaltet ist, dass eine Einspeisung und/oder ein Abgriff von X-Kern-Signalen über die elektrischen Anschlusselemente (12a-d) eines Paares aus zwei benachbarten, insbesondere in Umfangsrichtung der Volumenspule benachbarten, Anschlusseinrichtungen (11a-d) von insbesondere insgesamt vier Anschlusseinrichtungen (11a-d) erfolgen kann, insbesondere, wobei die Spulenanordnung (1) derart ausgestaltet ist, dass die sich im elektrisch verbindenden Zustand der Verbindungselemente (3a-h) ergebende Volumenspule und/oder Leiterschleifenanordnung über die beiden benachbarten Anschlusseinrichtungen (11a-d) in einem Quadraturbetrieb mit einer hochfrequenten Wechselspannung, deren Frequenz sich von der $^{1}$H-Kern-Resonanzfrequenz unterscheidet und bevorzugt einer X-Kern-Resonanzfrequenz entspricht, gespeist werden kann, um zu bewirken, dass die Volumenspule oder jede Leiterschleife (20a-c) der Leiterschleifenanordnung ein hochfrequentes elektromagnetisches Wechselfeld mit einer X-Kern-Resonanzfrequenz abstrahlt.

10. Spulenanordnung (1) nach einem der Ansprüche 7 bis 9, **dadurch gekennzeichnet, dass** in der Mitte einer Dipolantenne (2a-d) zwischen den beiden Polen ein Dipol-Kondensator (13a-d) vorgesehen ist, der insbesondere Teil der Anschlusseinrichtung (11a-d) ist, wobei der Dipol-Kondensator (13a-d) bevorzugt eine Kapazität von 0 pF aufweist.

11. Spulenanordnung (1) nach Anspruch 10, **dadurch gekennzeichnet, dass** der Dipol-Kondensator (13a-d) ausgebildet ist, insbesondere eine geeignete Kapazität aufweist, um die Spulenanordnung (1) auf die erste Frequenz abzustimmen, insbesondere feinabzustimmen.

12. Spulenanordnung (1) nach einem der Ansprüche 7 bis 11, **dadurch gekennzeichnet, dass** ein insbesondere parallel zu dem Dipol-Kondensator (13a-d) geschaltetes Koppelelement (14a-d) vorgesehen ist, das mit den beiden Polen der Dipolantenne (2a-d) elektrisch verbunden ist und diese überbrückt, und das Koppelelement (14a-d) ausgebildet ist, um von einem elektrisch verbindenden in einen elektrisch trennenden Zustand überführt zu werden, und umgekehrt, wobei das Koppelelement (14a-d) bevorzugt Teil der Anschlusseinrichtung (11a-d) ist,

insbesondere, wobei das Koppelelement (14a-d) einen Koppelelement-Sperrkreis (15a-d) umfasst, der automatisch sperrt, wenn eine hochfrequente Wechselspannung mit einer der Sperrfrequenz des Koppelelement-Sperrkreises (15a-d) entsprechenden Frequenz an die Spulenanordnung (1), insbesondere an die elektrischen Anschlusselemente (12a-d) der Anschlusseinrichtung (11a-d) der entsprechenden Dipolantenne (2a-d), angelegt wird, wobei die Sperrfrequenz des Koppelelement-Sperrkreises (15a-d) insbesondere der ersten Frequenz entspricht,

bevorzugt, wobei zumindest einer der Koppelelement-Sperrkreise (15a-d) eine Koppelelement-Spule (16a-d) und einen Koppelelement-Kondensator (17a-d) umfasst, die parallel geschaltet sind, wobei die Koppelelement-Spule (16a-d) bevorzugt eine Induktivität im Bereich von 38 bis 41 nH, besonders bevorzugt im Bereich von 39 bis 40 nH, insbesondere von 39 nH oder 40 nH aufweist und/oder der Koppelelement-Kondensator (17a-d) bevorzugt eine Kapazität im Bereich von 6 bis 8 pF, insbesondere von 6,8 pF aufweist,
insbesondere, wobei zumindest ein Koppelelement (14a-d) einen zweiten Koppelelement-Kondensator (18a-d) umfasst, der mit dem Koppelelement-Sperrkreis (15a-d) des Koppelelements (14a-d) in Reihe geschaltet ist, wobei der zweite Koppelelement-Kondensator (18a-d) bevorzugt eine Kapazität im Bereich von 20 bis 110 pF, besonders bevorzugt im Bereich von 33 bis 100 pF, insbesondere von 33 pF, 95 pF oder 100 pF aufweist,
bevorzugt, wobei der zweite Koppelelement-Kondensator (18a-d) ausgebildet ist, insbesondere eine geeignete Kapazität aufweist, um die Spulenanordnung (1) auf die zweite Frequenz abzustimmen, insbesondere feinabzustimmen, und/oder einen Kurzschluss bei der zweiten Frequenz auszulösen, und/oder dass die Dipolantennenanordnung (2) vier Dipolantennen (2a-d) umfasst, wobei die zweiten Koppelelement-Kondensatoren (18a-d) der Koppelelemente (14a-d) der Anschlusseinrichtungen (11a-d) zweier benachbarten Dipolantennen (2a-d), insbesondere zweier in Umfangsrichtung der Volumenspule benachbarten Dipolantennen (2a-d) jeweils eine gleiche Kapazität von insbesondere 33 pF aufweisen und die zweiten Koppelelement-Kondensatoren (17a-d) der beiden verbleibenden Dipolantennen (2a-d) ebenfalls eine gleiche Kapazität von insbesondere 95 pF oder 100 pF aufweisen.

13. Spulenanordnung (1) nach Anspruch 3 oder einem der Ansprüche 4 bis 12, sofern auf Anspruch 3 rückbezogen, **dadurch gekennzeichnet, dass** sich die axialen Enden des stabförmigen Basiselements (8a-d) zumindest einer Dipolantenne (2a-d) jeweils unter Zwischenschaltung eines Anschlussstellen-Kondensators (10a-h) an das jeweilige Leiterbahnsegment (9a-h) der Dipolantenne (2a-d) anschließen, wobei der Anschlussstellen-Kondensator (10a-h) bevorzugt eine Kapazität im Bereich von 10 bis 40 pF, besonders bevorzugt im Bereich von 18 bis 32 pF, insbesondere von 18 pF oder 32 pF aufweist.

14. Spulenanordnung (1) nach Anspruch 13, **dadurch gekennzeichnet, dass** der Anschlussstellen-Kondensator (10a-h) ausgebildet ist, insbesondere eine geeignete Kapazität aufweist, um die Spulenanordnung (1) auf die zweite Frequenz abzustimmen, insbesondere feinabzustimmen.

15. Spulenanordnung (1) nach einem der vorhergehenden Ansprüche, sofern auf Anspruch 2 rückbezogen, **dadurch gekennzeichnet, dass** die Spulenanordnung (1) insbesondere mittels der zweiten Verbindungselement-Kondensatoren (7a-h) und/oder der zweiten Koppelelement-Kondensatoren (18a-d) und/oder der Dipol-Kondensatoren (13a-d) und/oder der Anschlussstellen-Kondensatoren (10a-h) derart abgestimmt ist, dass jede Dipolantenne (2a-d) im elektrisch trennenden Zustand der Verbindungselemente (3a-h) und insbesondere der Koppelelemente (14a-d) ein hochfrequentes, elektromagnetisches Wechselfeld mit einer $^{1}$H-Kern-Resonanzfrequenz abstrahlen und/oder aufnehmen kann, und die sich im elektrisch verbindenden Zustand der Verbindungselemente (3a-h) und insbesondere der Koppelelemente (14a-d) ergebende Volumenspule und/oder jede Leiterschleife (20a-c) der sich im elektrisch verbindenden Zustand der Verbindungselemente (3a-h) und insbesondere der Koppelelemente (14a-d) ergebenden Leiterschleifenanordnung ein hochfrequentes, elektromagnetisches Wechselfeld mit einer X-Kern-Resonanzfrequenz abstrahlen und/oder aufnehmen kann, und/oder dass jede Leiterschleife (20a-c) der sich im elektrisch verbindenden Zustand der Verbindungselemente (3a-h) und insbesondere der Koppelelemente (14a-d) ergebenden Leiterschleifenanordnung zumindest durch Teile zweier benachbarter Dipolantennen (2a-d) gebildet wird, bevorzugt, wobei zumindest ein Teil einer Dipolantenne (2a-d), insbesondere, sofern vorhanden, zumindest deren stabförmiges Basiselement (8a-d), einen Teil zweier benachbarter Leiterschleifen (20a-c) bildet und bevorzugt die Kapazitätswerte der Verbindungselement-Kondensatoren (6a-h) und/oder der zweiten Verbindungselement-Kondensatoren (7a-h) und/oder der Koppelelement-Kondensatoren (17a-d) und/oder der zweiten Koppelelement-Kondensatoren (18a-d) und/oder der Dipol-Kondensatoren (13a-d) und/oder der Anschlussstellen-Kondensatoren (10a-h) und/oder weiterer Kondensatoren innerhalb der Leiterschleifenanordnung derart gewählt sind, dass benachbarte Leiterschleifen (20a-c) entkoppelt sind.

16. MR-System, insbesondere MRT-, bevorzugt Hochfeld-/Ultrahochfeld-MRT-, und/oder MRS-, Hochfeld-/Ultrahochfeld-MRS-System, mit einer Spulenanordnung (1) nach einem der vorhergehenden Ansprüche.

17. Verwendung einer Spulenanordnung (1) nach einem der Ansprüche 1 bis 15 als Hochfrequenz-Sende- und/oder Empfangsspule bei der Magnetresonanztomographie, insbesondere Hochfeld-/Ultrahochfeld-Magnetresonanztomographie und/oder Magnetresonanzspektroskopie, insbesondere Hochfeld-/Ultrahochfeld-Magnetresonanzspektroskopie.

## Claims

1. Coil arrangement (1) for use as a transmitter and/or reception coil in an MR system, in particular an MRI and/or MRS system, which coil arrangement (1) comprises a dipole antenna arrangement (2) with a plurality of dipole antennas (2a-d) connected to one another via connecting elements (3ah), the connecting elements (3a-h) being designed to be transferred from an electrically connecting state to an electrically disconnecting state, and vice versa, and the arrangement being made in such a manner that the dipole antennas (2a-d) in the electrically connecting state of the connecting elements (3a-h) form at least a part of a preferably cylindrical volume coil and/or a conductor loop arrangement, in particular a flat conductor loop arrangement, of the coil arrangement (1) comprising at least one conductor loop (20a-c), **wherein** the connecting elements (3a-h) comprise connecting element blocking circuits (4a-h), which automatically block when a high-frequency AC voltage having a frequency corresponding to the blocking frequency of the connecting element blocking circuits (4a-h) is applied to the coil arrangement (1), wherein the coil arrangement (1) is designed in such a way that the dipole antennas (2a-d) radiate and/or receive a high-frequency electromagnetic alternating field with a first frequency, in particular corresponding to the blocking frequency, when the connecting elements (3a-h) are in the electrically disconnecting state, and **in that** the volume coil resulting in the electrically connecting state of the connecting elements (3a-h) and/or each conductor loop (20a-c) of the conductor loop arrangement resulting in the electrically connecting state of the connecting elements (3a-h) radiate and/or receive a high-frequency, electromagnetic alternating field with a second frequency different from the first.

2. Coil arrangement (1) according to claim 1, **characterized in that** the first frequency is a $^{1}H$-core resonant frequency and the second frequency is an X-core resonant frequency, in particular a $^{31}P$-core or $^{23}Na$-core resonant frequency.

3. Coil arrangement (1) according to claims 1 or 2, **characterized in that** the dipole antennas (2a-d) each comprise a rod-shaped base element (8a-d), at the axially opposite ends of which a conductor path segment (9a-h), in particular in the form of a ring segment, adjoins respectively, the axial ends of the rod-shaped base element (8a-d) adjoining the respective conductor path segment (9a-h) in particular centrally, and the conductor path segments (9ah) of the dipole antennas (2a-d) preferably being of the same size, in particular having the same arc length, in particular, wherein

   the rod-shaped base elements (8a-d) of the dipole antennas (2a-d) are arranged at least substantially parallel to one another and/or the rod-shaped base elements (8a-d) of the dipole antennas (2a-d) are arranged uniformly spaced apart from one another in the circumferential direction of the volume coil or in the longitudinal direction of the conductor loop arrangement and/or the length of the rod-shaped base elements (8a-d) of the dipole antennas (2a-d), the height of the volume coil or the width of the conductor loop arrangement is in the range from 25 to 30 cm, in particular is 25 cm or 28 cm, and/ or
   the conductor path segments (9a-h) of the dipole antennas (2a-d) are connected to one another via the connecting elements (3a-h) to form two conductor paths (9), in particular two annularly closed conductor paths (9), and in particular the number of connecting elements (3a-h) per conductor path (9) is less by one than the number of dipole antennas (2a-d) or corresponds to the number of dipole antennas (2a-d), and/or the diameter of the annularly closed conductor paths (9) or of the volume coil is in the range from 25 to 30 cm, in particular is 26 cm.

4. Coil arrangement (1) according to any of the preceding claims, **characterized in that** at least one of the connecting element blocking circuits (4ah) comprises a connecting element coil (5a-h) and a connecting element capacitor (6a-h), which are connected in parallel, wherein the connecting element coil (5a-h) preferably has an inductance in the range from 38 to 41 nH, particularly preferably in the range from 39 to 40 nH, in particular of 39 nH or 40 nH, and/or the connecting element capacitor (6a-h) preferably has a capacitance in the range from 6 to 8 pF, in particular of 6.8 pF.

5. Coil arrangement (1) according to claim 4, **characterized in that** at least one connecting element (3a-h) comprises a second connecting element capacitor (7a-h) which is connected in series with the connecting element blocking circuit (4a-h), the second connecting element capacitor (7a-h) preferably having a capacitance in the range from 5 to 50 pF, in particular preferably in the range from 8.2 to 40 pF, in particular of 8.2 pF or 40 pF.

6. Coil arrangement (1) according to claim 5, **characterized in that** the second connecting element capacitor (7a-h) is designed, in particular has a suitable capacitance, to tune, in particular fine-tune, the coil arrangement (1) to the second frequency.

7. Coil arrangement (1) according to claim3 or one of claims 4 to 6, if referred back to claim 3, **characterized in that** the rod-shaped base elements (8a-d) of the dipole antennas (2a-d) are each preferably separated centrally to form two poles of the respective dipole antenna (2a-d).

**8.** Coil arrangement (1) according to claim 7, **characterized in that** the rod-shaped base elements (8a-d) of the dipole antennas (2a-d) are each provided with a junction device (11a-d) for connection to an AC voltage supply and/or scanning device, which comprises electrical junction elements (12a-d) connected to the two poles of the dipole antenna (2a-d).

**9.** Coil arrangement (1) according to claim 2 and claim 8, **characterized in that** the coil arrangement (1) is designed in such a way that a feed and/or a tap of $^{1}$H-core signals and/or X-core signals can take place via the electrical junction elements (12a-d) of at least one junction device (11a-d), in particular of all junction devices (11a-d), preferably, wherein

the coil arrangement (1) is designed in such a way that a feed and/or a tap of X-core signals can take place via the electrical junction elements (12a-d) of a pair of two adjacent, in particular adjacent in the circumferential direction of the volume coil, junction devices (11a-d) of, in particular, a total of four junction devices (11a-d), in particular, wherein

the coil arrangement (1) is designed in such a way that the volume coil and/or conductor loop arrangement resulting in the electrically connecting state of the connecting elements (3a-h) can be fed via the two adjacent junction devices (11a-d) in quadrature mode with a high-frequency AC voltage, whose frequency differs from the $^{1}$H-core resonant frequency and preferably corresponds to an X-core resonant frequency in order to cause the volume coil or each conductor loop (20a-c) of the conductor loop arrangement to radiate a high-frequency alternating electromagnetic field having an X-core resonant frequency.

**10.** Coil arrangement (1) according to one of claims 7 to 9, **characterized in that** a dipole capacitor (13a-d) is provided in the center of a dipole antenna (2a-d) between the two poles, which dipole capacitor (13a-d) is in particular part of the junction device (11a-d), the dipole capacitor (13a-d) preferably having a capacitance of 0 pF.

**11.** Coil arrangement (1) according to claim 10, **characterized in that** the dipole capacitor (13a-d) is designed, in particular has a suitable capacitance, to tune, in particular fine-tune, the coil arrangement (1) to the first frequency.

**12.** Coil arrangement (1) according to any one of claims 7 to 11, **characterized in that** a coupling element (14a-d) is provided, in particular connected in parallel with the dipole capacitor (13a-d), which is electrically connected to and bridges the two poles of the dipole antenna (2a-d), and the coupling element (14a-d) is designed to be transferred from an electrically connecting state to an electrically disconnecting state, and vice versa, the coupling element (14a-d) preferably being part of the junction device (11a-d), in particular, wherein

the coupling element (14a-d) comprises a coupling element blocking circuit (15a-d), which automatically blocks when a high-frequency AC voltage with a frequency corresponding to the blocking frequency of the coupling element blocking circuit (15a-d) is applied to the coil arrangement (1), in particular to the electrical junction elements (12a-d) of the junction device (11a-d) of the corresponding dipole antenna (2a-d), wherein the blocking frequency of the coupling element blocking circuit (15a-d) corresponds in particular to the first frequency, preferably, wherein

at least one of the coupling element blocking circuits (15a-d) comprises a coupling element coil (16a-d) and a coupling element capacitor (17a-d), which are connected in parallel, wherein the coupling element coil (16a-d) preferably has an inductance in the range from 38 to 41 nH, particularly preferably in the range from 39 to 40 nH, in particular of 39 nH or 40 nH, and/or the coupling element capacitor (17a-d) preferably has a capacitance in the range from 6 to 8 pF, in particular of 6.8 pF, in particular, wherein

at least one coupling element (14a-d) comprises a second coupling element capacitor (18a-d) which is connected in series with the coupling element blocking circuit (15a-d) of the coupling element (14a-d), the second coupling element capacitor (18a-d) preferably having a capacitance in the range from 20 to 110 pF, in particular preferably in the range from 33 to 100 pF, in particular of 33 pF, 95 pF or 100 pF, preferably, wherein

the second coupling element capacitor (18a-d) is designed, in particular has a suitable capacitance, to tune, in particular fine-tune, the coil arrangement (1) to the second frequency and/or to trigger a short circuit at the second frequency, and/ or

that the dipole antenna arrangement (2) comprises four dipole antennas (2a-d), wherein the second coupling element capacitors (18a-d) of the coupling elements (14a-d) of the junction devices (11a-d) of two adjacent dipole antennas (2a-d), in particular of two dipole antennas (2a-d) adjacent in the circumferential direction of the volume coil, each have an equal capacitance of in particular 33 pF, and the second coupling element capacitors (17a-d) of the two remaining dipole antennas (2a-d) likewise have an equal capacitance of in particular 95 pF or 100 pF.

**13.** Coil arrangement (1) according to claim 3 or one of claims 4 to 12, if referred back to claim 3, **characterized in that** the axial ends of the rod-shaped base element (8a-d) of at least one dipole antenna (2a-d) each connect to the respective conductor path segment (9a-h) of the dipole antenna (2a-d) with the interposition of a junction point capacitor (10a-h), the junction point capacitor (10a-h) preferably having a capacitance in the range from 10 to 40 pF, particularly preferably in the range from 18 to 32 pF, in particular of 18 pF or 32 pF.

**14.** Coil arrangement (1) according to claim 13, **characterized in that** the junction point capacitor (10a-h) is designed, in particular has a suitable capacitance, to tune, in particular fine-tune, the coil arrangement (1) to the second frequency.

**15.** Coil arrangement (1) according to any of the preceding claims, if referred back to claim 2, **characterized in that** the coil arrangement (1) is tuned, in particular by means of the second connecting element capacitors (7a-h) and/or the second coupling element capacitors (18a-d) and/or the dipole capacitors (13a-d) and/or the junction point capacitors (10a-h) such, that each dipole antenna (2a-d) in the electrically disconnecting state of the connecting elements (3a-h) and in particular of the coupling elements (14a-d) can radiate and/or receive a high-frequency electromagnetic alternating field with a $^{1}$H-core resonant frequency, and the volume coil resulting in the electrically connecting state of the connecting elements (3a-h) and in particular of the coupling elements (14a-d) and/or each conductor loop (20a-c) of the conductor loop arrangement resulting in the electrically connecting state of the connecting elements (3a-h) and in particular of the coupling elements (14a-d) can radiate and/or receive a high-frequency electromagnetic alternating field with an X-core resonant frequency, and/ or that

each conductor loop (20a-c) of the conductor loop arrangement resulting in the electrically connecting state of the connecting elements (3a-h) and in particular of the coupling elements (14a-d) is formed at least by parts of two adjacent dipole antennas (2a-d), preferably, wherein
at least a part of a dipole antenna (2a-d), in particular, if present, at least its rod-shaped base element (8a-d), forms part of two adjacent conductor loops (20a-c) and preferably the capacitance values of the connecting element capacitors (6a-h) and/or of the second connecting element capacitors (7a-h) and/or of the coupling element capacitors (17a-d) and/or of the second coupling element capacitors (18a-d) and/or of the dipole capacitors (13a-d) and/or of the junction point capacitors (1 0a-h) and/or of further capacitors within the conductor loop arrangement are selected in such a manner that adjacent conductor loops (20a-c) are decoupled.

**16.** MR system, in particular MRI, preferably high-field/ultra-high-field MRI, and/or MRS, high-field/ultra-high-field MRS system, having a coil arrangement (1) according to one of the preceding claims.

**17.** Use of a coil arrangement (1) according to any one of claims 1 to 15 as a high-frequency transmitter and/or reception coil in magnetic resonance imaging, in particular high-field/ultra-high-field magnetic resonance imaging and/or magnetic resonance spectroscopy, in particular high-field/ultra-high-field magnetic resonance spectroscopy.

**Revendications**

**1.** Dispositif à bobines (1) destiné à être utilisé comme bobine d'émission et/ou de réception dans un système RM, en particulier un système IRM et/ou SMR, qui comprend un dispositif d'antennes dipôles (2) avec plusieurs antennes dipôles (2a-d) reliées entre elles par des éléments de liaison (3a-h), les éléments de liaison (3a-h) étant conçus pour passer d'un état de connexion électrique à un état de séparation électrique, et inversement, et l'agencement étant tel que les antennes dipôles (2a-d), dans l'état de connexion électrique des éléments de connexion (3a-h), forment au moins une partie d'une bobine volumique de préférence cylindrique et/ou un agencement de boucles conductrices, en particulier plat, comprenant au moins une boucle conductrice (20a-c) de l'agencement de bobines (1) , les éléments de liaison (3a-h) comprenant des circuits de blocage d'éléments de liaison (4a-h) qui se bloquent automatiquement lorsqu'une tension alternative à haute fréquence d'une fréquence correspondant à la fréquence de blocage des circuits de blocage d'éléments de liaison (4a-h) est appliquée au dispositif à bobines (1), le dispositif à bobines (1) est conçu de telle sorte que les antennes dipôles (2a-d), lorsque les éléments de liaison (3a-h) sont électriquement séparés, émettent et/ou captent un champ électromagnétique alternatif à haute fréquence avec une première fréquence correspondant à la fréquence de blocage, et que la bobine de volume résultant des éléments de liaison (3a-h) et/ou chaque boucle conductrice (20a-c) de l'antenne dipôle (2a-d) dans l'état de connexion électrique des éléments de liaison (3a-h) (3a-h) et/ou chaque boucle conductrice (20a-c) de l'agencement de boucles conductrices résultant de l'état de connexion électrique des éléments de connexion (3a-h) émettent et/ou reçoivent un champ électromagnétique alternatif à haute fréquence avec une deuxième fréquence différente de la première.

**2.** Dispositif à bobines (1) selon la revendication 1, **caractérisé en ce que** la première fréquence est une fréquence de résonance du noyau H et la deuxième fréquence est une fréquence de résonance du noyau X, en particulier une fréquence de résonance du noyau P ou du noyau Na.

3. Dispositif à bobines (1) selon la revendication 1 ou 2, **caractérisé en ce que** les antennes dipôles (2a-d) comprennent chacune un élément de base en forme de tige (8a-d) aux extrémités axialement opposées duquel se raccorde respectivement un segment de piste conductrice (9a-h) en particulier en forme de segment annulaire, les extrémités axiales de l'élément de base en forme de tige (8a-d) sont reliées en particulier au centre du segment de piste conductrice (9a-h) correspondant, et les segments de piste conductrice (9a-h) des antennes dipôles (2a-d) étant de préférence de même taille, en particulier présentant la même longueur d'arc, en particulier les éléments de base en forme de tige (8a-d) des antennes dipôles (2a-d) sont au moins essentiellement parallèles les uns aux autres et/ou les éléments de base en forme de tige (8a-d) des antennes dipôles (2a-d) sont disposés à intervalles réguliers les uns par rapport aux autres dans le sens circonférentiel de la bobine volumique ou dans le sens longitudinal de l'agencement de boucles conductrices et/ou la longueur des éléments de base en forme de tige (8a-d) des antennes dipôles (2a-d), la hauteur de la bobine volumique ou la largeur de l'agencement de boucles conductrices est comprise entre 25 et 30 cm, en particulier 25 cm ou 28 cm, et/ou les segments de piste conductrice (9a-h) des antennes dipôles (2a-d) sont reliés entre eux par les éléments de liaison (3a-h) en formant deux pistes conductrices (9), en particulier deux pistes conductrices (9) fermées en anneau, et en particulier le nombre d'éléments de liaison (3a-h) par piste conductrice (9) est inférieur d'une unité au nombre d'antennes dipôles (2a-d) ou correspond au nombre d'antennes dipôles (2a-d), et/ou le diamètre des pistes conductrices fermées en anneau (9) ou de la bobine volumineuse est compris entre 25 et 30 cm, en particulier 26 cm.

4. Dispositif à bobines (1) selon l'une des revendications précédentes, **caractérisé en ce qu'**au moins l'un des circuits de blocage d'éléments de connexion (4a-h) comprend une bobine d'élément de connexion (5a-h) et un condensateur d'élément de connexion (6a-h) qui sont montés en parallèle, la bobine d'élément de connexion (5a-h) présente de préférence une inductance comprise entre 38 et 41 nH, de préférence comprise entre 39 et 40 nH, en particulier de 39 nH ou 40 nH, et/ou le condensateur d'élément de liaison (6ah) présente de préférence une capacité comprise entre 6 et 8 pF, en particulier de 6,8 pF.

5. Dispositif à bobines (1) selon la revendication 4, **caractérisé en ce qu'**au moins un élément de connexion (3a-h) comprend un deuxième condensateur d'élément de connexion (7a-h) qui est monté en série avec le circuit de blocage d'élément de connexion (4a-h), le deuxième condensateur d'élément de connexion (7a-h) présente de préférence une capacité comprise entre 5 et 50 pF, de préférence comprise entre 8,2 et 40 pF, en particulier de 8,2 pF ou 40 pF.

6. Dispositif à bobines (1) selon la revendication 5, **caractérisé en ce que** le deuxième condensateur d'élément de connexion (7a-h) est conçu, en particulier présente une capacité appropriée, pour accorder, en particulier régler finement, le dispositif à bobines (1) sur la deuxième fréquence.

7. Dispositif à bobines (1) selon la revendication 3 ou l'une des revendications 4 à 6, dans la mesure où elles se réfèrent à la revendication 3, **caractérisé en ce que** les éléments de base en forme de tige (8a-d) des antennes dipôles (2a-d) sont séparés de préférence au centre en formant deux pôles de l'antenne dipôle respective (2a-d).

8. Dispositif à bobines (1) selon la revendication 7, **caractérisé en ce que** les éléments de base en forme de tige (8a-d) des antennes dipôles (2a-d) sont chacun pourvus d'un dispositif de raccordement (11a-d) destiné à être relié à un dispositif d'alimentation en tension alternative et/ou à un dispositif de réception, qui comprend des éléments de raccordement électrique (12a-d) reliés aux deux pôles de l'antenne dipôle (2a-d).

9. Dispositif à bobines (1) selon la revendication 2 et la revendication 8, **caractérisé en ce que** le dispositif à bobines (1) est conçu de telle sorte qu'une alimentation et/ou une prise de signaux à noyau $^{1}$H et/ou de signaux à noyau X via les éléments de raccordement électrique (12a-d) d'au moins un dispositif de raccordement (11a-d), en particulier de tous les dispositifs de raccordement (11a-d), de préférence, l'agencement de bobines (1) est conçu de telle sorte qu'une alimentation et/ou une prise de signaux X-noyau via les éléments de raccordement électriques (12a-d) d'une paire de deux dispositifs de raccordement (11a-d) adjacents, en particulier adjacents dans le sens circonférentiel de la bobine volumique, de quatre dispositifs de raccordement (11a-d) au total, peut avoir lieu, en particulier, l'agencement de bobines (1) étant conçu de telle sorte que la bobine de volume et/ou l'agencement de boucles conductrices résultant de l'état de connexion électrique des éléments de connexion (3a-h) peut être alimenté, par l'intermédiaire des deux dispositifs de raccordement adjacents (11a-d), en quadrature avec une tension alternative à haute fréquence dont la fréquence se distingue de la fréquence de résonance $^{1}$H et correspond de préférence à une fréquence de résonance X, afin que la bobine volumique ou chaque boucle conductrice (20a-c) de l'agencement de boucles conductrices émette un champ électromagnétique alternatif à haute fréquence avec une fréquence de résonance X.

**10.** Dispositif à bobines (1) selon l'une des revendications 7 à 9, **caractérisé en ce qu'**au centre d'une antenne dipôle (2a-d) **caractérisé en ce qu'**un condensateur dipôle (13a-d) est prévu au centre d'une antenne dipôle (2a-d) entre les deux pôles, lequel fait notamment partie du dispositif de raccordement (11a-d), le condensateur dipôle (13a-d) présentant de préférence une capacité de 0 pF.

**11.** Dispositif à bobines (1) selon la revendication 10, **caractérisé en ce que** le condensateur dipôle (13a-d) est conçu, en particulier présente une capacité appropriée, pour accorder, en particulier régler finement, le dispositif à bobines (1) sur la première fréquence.

**12.** Dispositif à bobines (1) selon l'une des revendications 7 à 11, **caractérisé en ce qu'**un élément de couplage (14a-d) est prévu, notamment en parallèle avec le condensateur dipôle (13a-d), qui est relié électriquement aux deux pôles de l'antenne dipôle (2a-d) et les relie entre eux, et l'élément de couplage (14a-d) est conçu pour être commuté d'un état de connexion électrique à un état de séparation électrique, et inversement, l'élément de couplage (14a-d) faisant de préférence partie du dispositif de raccordement (11ad), en particulier l'élément de couplage (14a-d) comprend un circuit de blocage d'élément de couplage (15a-d) qui se bloque automatiquement lorsqu'une tension alternative à haute fréquence d'une fréquence correspondant à la fréquence de blocage du circuit de blocage d'élément de couplage (15a-d) est appliquée à l'ensemble de bobines (1), en particulier aux éléments de raccordement électrique (12a-d) du dispositif de raccordement (11a-d) de l'antenne dipôle correspondante (2a-d), la fréquence de blocage du circuit de blocage de l'élément de couplage (15a-d) correspondant en particulier à la première fréquence,
de préférence, au moins l'un des circuits de blocage d'élément de couplage (15a-d) comprenant une bobine d'élément de couplage (16a-d) et un condensateur d'élément de couplage (17a-d) qui sont montés en parallèle, la bobine d'élément de couplage (16a-d) présente de préférence une inductance comprise entre 38 et 41 nH, de préférence comprise entre 39 et 40 nH, en particulier de 39 nH ou 40 nH, et/ou le condensateur d'élément de couplage (17a-d) présente de préférence une capacité comprise entre 6 et 8 pF, en particulier de 6,8 pF, en particulier, où le deuxième condensateur d'élément de couplage (18a-d) est conçu, en particulier présente une capacité appropriée, pour accorder l'agencement de bobines (1) sur la deuxième fréquence, en particulier pour l'accorder avec précision, et/ou pour déclencher un court-circuit à la deuxième fréquence, et/ou que l'agencement d'antennes dipôles (2) comprend quatre antennes dipôles (2a-d), les seconds condensateurs d'éléments de couplage (18a-d) des éléments de couplage (14a-d) des dispositifs de raccordement (11a-d) de deux antennes dipôles voisines (2a-d), en particulier de deux antennes dipôles voisines dans le sens circonférentiel de la bobine volumique (2a-d) présentent chacune une capacité identique, en particulier de 33 pF, et les seconds condensateurs d'éléments de couplage (17a-d) des deux antennes dipôles restantes (2a-d) présentent également une capacité identique, en particulier de 95 pF ou 100 pF.

**13.** Dispositif à bobines (1) selon la revendication 3 ou l'une des revendications 4 à 12, dans la mesure où elles se réfèrent à la revendication 3, **caractérisé en ce que** les extrémités axiales de l'élément de base en forme de tige (8a **caractérisé en ce que** les extrémités axiales de l'élément de base en forme de tige (8a-d) d'au moins une antenne dipôle (2a-d) sont chacune raccordées au segment de piste conductrice (9a-h) correspondant de l'antenne dipôle (2a-d) avec interposition d'un condensateur de point de connexion (10ah), le condensateur de point de connexion (10a-h) présente de préférence une capacité comprise entre 10 et 40 pF, de préférence entre 18 et 32 pF, en particulier de 18 pF ou 32 pF.

**14.** Dispositif à bobines (1) selon la revendication 13, **caractérisé en ce que** le condensateur de point de connexion (10a-h) est conçu, en particulier présente une capacité appropriée, pour accorder, en particulier pour accorder avec précision, le dispositif à bobines (1) sur la deuxième fréquence.

**15.** Dispositif à bobines (1) selon l'une des revendications précédentes, dans la mesure où il est fait référence à la revendication 2, **caractérisé en ce que** le dispositif à bobines (1) est notamment adapté, au moyen des seconds condensateurs d'éléments de liaison (7a-h) et/ou des seconds condensateurs d'éléments de couplage (18a-d) et/ou des condensateurs dipolaires (13a-d) et/ou des condensateurs de points de connexion (10a-h) de telle sorte que chaque antenne dipôle (2a-d), lorsque les éléments de liaison (3a-h) et en particulier les éléments de couplage (14a-d) sont électriquement séparés, peut émettre et/ou recevoir un champ électromagnétique alternatif à haute fréquence avec une fréquence de résonance $^{1}$H, et la bobine de volume et/ou chaque boucle conductrice (20a-c) résultant de la disposition en boucle conductrice des éléments de connexion (3a-h) et en particulier des éléments de couplage (14a-d) à l'état de connexion électrique des éléments de connexion (3a-h) et en particulier des éléments de couplage (14a-d) émettent un champ électromagnétique alternatif à haute fréquence avec une résonance X-core. (3a-h) et en particulier des éléments de couplage (14a-d) peut émettre et/ou recevoir un champ électromagnétique alternatif à haute fréquence avec une fréquence de résonance X-noyau, et/ou que chaque boucle conductrice (20a-c) de la disposition de boucles conductrices résultant de l'état de connexion électrique des éléments de connexion (3a-h) et en particulier des éléments de couplage (14a-d), est formée au moins par des parties de deux antennes dipôles voisines (2a-d), de préférence au moins une partie d'une antenne dipôle (2a-d), en particulier, si elle est présente, au moins son élément de base en forme de tige (8a-d), formant une partie de deux boucles conductrices voisines (20a-c) et de préférence les valeurs de capacité des condensateurs d'éléments de liaison (6a-h) et/ou des seconds condensateurs d'éléments de liaison (7a-h) et/ou des condensateurs d'éléments de couplage (17a-d) et/ou des seconds condensateurs d'éléments de couplage (18a-d) et/ou des condensateurs dipôles (13a-d) et/ou des condensateurs de point de connexion (10a-h) et/ou d'autres condensateurs à l'intérieur de l'agencement de boucles conductrices sont choisies de telle sorte que les boucles conductrices voisines (20a-c) sont découplées.

**16.** Système RM, en particulier IRM, de préférence IRM haute/ultra-haute fréquence, et/ous SRM, SRS haute/ultra-haute fréquence, avec un agencement de bobines (1) selon l'une des revendications précédentes.

**17.** Utilisation d'un agencement de bobines (1) selon l'une des revendications 1 à 15 comme bobine d'émission et/ou de réception à haute fréquence dans la tomographie par résonance magnétique, en particulier la tomographie par résonance magnétique à champ élevé/ultra-élevé et/ou la spectroscopie par résonance magnétique, en particulier la spectroscopie par résonance magnétique à champ élevé/ultra-élevé.

Figur 1

1
3b
9
2c
10c
3c
2b
10b
3a
2a
10a
2d
10d
3d
11b
11c
2
11a
11d
10f
19
10g
3e
10e
3f
3g
10h
10e
9
3h

2a
2b
2c
2d
9a
9b
9c
9d
8a
8b
8c
8d
19
9e
9f
9g
9h

Fig. 2

2a
2b
2c
2d
3a
3b
3c
3d
4a
4b
4c
4d
5a
5b
5c
5d
6a
6b
6c
6d
7a
7b
7c
7d
8a
8b
8c
8d
9a
9b
9c
9d
10a
10b
10c
10d
11a
11b
11c
11d
12a
12b
12c
12d
13a
13b
13c
13d
14a
14b
14c
14d
15a
15b
15c
15d
16a
16b
16c
16d
17a
17b
17c
17d
18a
18b
18c
18d
21a
21b
21c
21d
10e
10f
10g
10h
3e
3f
3g
3h
4e
4f
4g
4h
5e
5f
5g
5h
6e
6f
6g
6h
7e
7f
7g
7h
9e
9f
9g
9h
20a
20b
20c

Fig. 3

Figur 4

S-Parameter [Magnitude in dB]

0
-2
-4
-6
-8
-10
-12
-14
-16
-18
50
100
150
200
250
300
350
400

Frequenz / MHz

Figur 5

Figur 6

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 2229865 A
- US 5462055 A
- US 20100253333 A1
- US 5202635 A


### In der Beschreibung aufgeführte Nicht-Patentliteratur


- **DETEKTIERUNG** ; **H-KERNEN** ; **A.J.E. RAAIJMAKERS**. Design of a radiative surface coil array element at 7T: the single-side adapted dipole antenna. *Magn. Reson. Med. Off. J. Soc. Magn. Reson. Med. Soc. Magn. Reson. Med.*, November 2011, vol. 66 (5), 1488-1497
- **HONG SUK-MIN** ; **PARK JOSHUA HAEKYUN** ; **WOO MYUNG-KYUN** ; **KIM YOUNG-BO** ; **CHO ZANG-HEE**. New design concept of monopole antenna array für UHF 7T MRI. *Magn. Reson. Med.*, July 2013, vol. 71 (5), 1944-1952
- **D.W. ALDERMANN** ; **D.M. GRANT**. An efficient decoupler coil design which reduces heating in conductive samples in superconducting spectrometers. *Magn. Reson.*, 1969, vol. 36 (3), 447-451
- **J.R. FITZSIMMONS** ; **B.L. BECK** ; **H. RALPH BROOKER**. Double resonant quadrature birdcage. *Magn. Reson. Med.*, 1993, vol. 30 (1), 107-114
- **A.M. HUDSON** ; **W. KÖCKENBERGER** ; **R.W. BOWTELL**. Dual resonant birdcage coils for 1H detected 13C microscopic imaging at 11.7T. *Magma N.Y.N*, June 2000, vol. 10 (2), 61-68
- **Y. DUAN** ; **B.S. PETERSON** ; **F. LIU** ; **T.R. BROWN** ; **T.S. IBRAHIM** ; **A. KANGARLU**. Computational and experimental optimization of a double-tuned 1H/31P four-ring birdcage head coil für MRS at 3T. *J.Magn. Reson. Imaging*, 2009, vol. 29 (1), 13-22
- **J. MURPHYBOESCH** ; **R. SRINIVASAN** ; **L. CARVAJAL** ; **T.R. BROWN**. Two Configurations of the Four-Ring Birdcage Coil for 1H Imaging and 1H-Decoupled 31P Spectroscopy of the Human Head. *J. Magn. Reson. B*, 1994, vol. 103 (2), 103-114
- **G.B. MATSON** ; **P. VERMATHEN** ; **T.C. HILL**. A parctical double-tuned 1H/31P quadrature birdcage headcoil optimized for 31P operation. *Magn. Reson. Med.*, 1999, vol. 42 (1), 173-182
- **C.-L. CHIN** ; **C. M. COLLINS** ; **S. LI** ; **B.J. DARDZINSKI** ; **M.B. SMITH**. BirdcageBuilder: Design of Specified-Geometry Birdcage Coils with Desired Current Pattern and Resonant Frequency. *Concepts Magn. Reson.*, June 2002, vol. 15 (2), 156-163
- **G. JANZEN**. Kurze Antennen. Entwurf und Berechnung verkürzter Sende- und Empfangsantennen. Kosmos Verlags GmbH, June 1989
- **A.L. PERRIER** ; **D. GRENIER** ; **N. RAVEL** ; **P. LITAUDON** ; **O. BEUF**. Capacitive approach to restore decoupling between channels for four-element MR coil array. *ELECTRONICS LETTERS*, 20 June 2013, vol. 49 (13)